# EUROPEAN PATENT SPECIFICATION

(11) **EP 4 479 083 B1**
(45) Date of publication and mention of the grant of the patent: **22.04.2026**
(21) Application number: 23705000.0
(22) Date of filing: 16.02.2023
(51) Int. Cl.: A61K 39/00, A61K 31/145, A61P 35/02

(54) **METHODS OF TREATMENT WITH CAR CELLS IN COMBINATION WITH S1P RECEPTOR MODULATORS**
BEHANDLUNGSVERFAHREN MIT CAR-ZELLEN IN KOMBINATION MIT S1P-REZEPTORMODULATOREN
PROCÉDÉS DE TRAITEMENT UTILISANT DES CELLULES CAR EN COMBINAISON AVEC DES MODULATEURS DU RÉCEPTEUR S1P

(30) Priority: 16.02.2022 EP 22305171; 27.05.2022 EP 22305781
(43) Date of publication of application: 25.12.2024
(73) Proprietor: Priothera SAS, 68300 Saint-Louis (FR)
(72) Inventor: DERTSCHNIG, Simone, 4054 Basel (CH); BOUSSO, Philippe, 75015 Paris (FR); BOULCH, Morgane, 75015 Paris (FR); OEHEN, Stephan, 4123 Allschwil (CH)
(74) Representative: Plasseraud IP
(86) International application number: PCT/EP2023/053857
(87) International publication number: WO 2023/156506

(56) References cited:
- WO-A1-2011/140170
- WO-A1-2014/128611
- WO-A1-2017/141243
- MARCUS A ET AL: "Redirected tumor-specific allogeneic T cells for universal treatment of cancer", BLOOD, vol. 118, no. 4, 28 July 2011 (2011-07-28), pages 975 - 983, XP002712636, ISSN: 0006-4971, DOI: 10.1182/BLOOD-2011-02-334284
- BRUDNO J N & KOCHENDORFER J N: "Toxicities of chimeric antigen receptor T cells: recognition and management", BLOOD, vol. 127, no. 26, 20 May 2016 (2016-05-20), pages 3321 - 3330, XP055547499, ISSN: 0006-4971, DOI: 10.1182/blood-2016-04-703751

## Description

### TECHNICAL FIELD

The present invention relates to the field of cellular immunotherapy and particularly to composition comprising CAR cells that express a chimeric antigen receptor molecule that binds a cancer-related antigen for use in combination therapy with a S1P receptor agonist in the treatment of hematological malignancies, in a subject in need thereof.

### BACKGROUND

Many patients with hematological malignancies are incurable with standard therapy. In addition, traditional treatment options often have serious side effects. Attempts have been made with cancer immunotherapies, however, several obstacles render this a very difficult goal to achieve clinical effectiveness. Although hundreds of so-called tumor antigens have been identified, these are generally derived from self and thus are poorly immunogenic.

The presence of naturally occurring tumor-reactive T cells has been thoroughly documented in the peripheral blood, and within the tumors, of cancer patients. However, although T cells recognize neoplastic cells, their presence is often insufficient to mediate clinical tumor regressions. Tumors employ a myriad of mechanisms to neutralize or evade immune attack, particularly T cell-mediated responses. These mechanisms include downregulation of MHC molecule expression, or disruption of the antigen processing and presentation machineries, among others.

Adoptive transfer of autologous immune cells, modified ex *vivo* to express chimeric antigen receptors (CARs) has emerged as a new therapeutic tool to circumvent some of these obstacles. Such approaches which rely on redirecting such immune effector cells to a suitable cell-surface molecule on cancer cells such as hematological malignancies. For example, in recent clinical trials, CAR-T cells targeting the CD19 molecule have demonstrated remarkable activity in the treatment of B cell leukemias and B cell lymphomas (Maude et al., 2014; Neelapu et al., 2017; Schuster et al., 2017; Park et al., 2018). Despite these promising results, circulating tumor cells have been found to strongly prevent CAR-T cell traffic to the bone marrow, hence representing a substantial hurdle for CAR-T cell mediated elimination of target tumor cells and persistence of CAR cells (M. Cazaux et al.; J Exp Med 6 May 2019; 216 (5): 1038-1049. doi: https://doi.org/10.1084/jem.20182375). Therefore, besides the ability for the chimeric antigen receptor on the genetically modified immune effector cells to recognize and destroy the targeted cells, a successful therapeutic immune effector cell therapy needs to have the ability to proliferate, persist, remain functionally active and traffic to the bone marrow over time, in order to survey for leukemic relapse.

Unfortunately, CAR-T cell therapy can also cause severe toxicities affecting different organs and limiting the success of the therapy (Brudno and Kochenderfer, Blood 127, 3321-3330 (2016)). CAR-T cells are highly activated upon tumor antigen recognition which triggers cytokine release. This local cytokine storm can further activate bystander immune cells, which release more inflammatory cytokines contributing to cytokine release syndrome (CRS) pathology which characterized by high systemic cytokine levels. Activated immune cells will migrate to peripheral sites causing a systemic inflammatory response in tissue (Lee et al. Blood 2014 124(2):188-195).

Even in the clinical trials with the most dramatic response rates, severe, life-threatening events have occurred in patients. Specifically, in the case of acute lymphoblastic leukemia/lymphoma (ALL/LBL) patients treated with CAR-T cell therapy, nearly all patients have at least some less severe toxicity manifestations while 20-50% of patients displayed severe supraphysiologic cytokine production and massive in-vivo T cell expansion. These toxic levels of systemic cytokine release and severe immune cell cross-activation in some patients result in the following toxicities: (1) cytokine-release syndrome (CRS), which is associated with supraphysiologic cytokine production and massive in vivo T cell expansion; (2) hemophagocytic lymphohistiocytosis and/or macrophage activation syndrome (MAS) defined as a severe hyperinflammatory syndrome characterized by CRS and combinations of elevated serum ferritin and hemophagocytosis, renal failure, liver enzymes, splenomegaly, pulmonary edema, and/or absence of **NK** cell activity; and (3) immune effector cell-associated neurotoxicity syndrome (ICANS), which is characterized by elevated cerebrospinal fluid cytokine levels and blood-brain barrier disruption (Sheth, V.S., Gauthier, J. Taming the beast: CRS and ICANS after CAR T-cell therapy for ALL. Bone Marrow Transplant 56, 552-566 (2021)).

Another challenge with CAR-T cell therapy is the loss of efficacy against hematological cancers, caused by e.g. inhibition and resistance in cancer cell malignancies, antigen escape, limited CAR persistence, poor CAR trafficking and tumor infiltration, and the immunosuppressive microenvironment. For example, although 70-90% of relapsed and/or refractory ALL patients show durable responses to CD19 targeted CAR-T cell therapy, recent follow-up data suggest development of a common disease resistance mechanism, including downregulation/loss of CD19 antigen in 30-70% of patients who have recurrent disease after treatment (Sterner, R.C., Sterner, R.M. CAR-T cell therapy: current limitations and potential strategies. Blood Cancer J. 11, 69 (2021)). There is thus an ongoing need for improved strategies for targeting hematological malignancies, in particular, new compositions and methods for improving CAR therapies are highly desirable.

### SUMMARY

Promoting sequestration of CAR-T cells into lymphoid tissues such as bone marrow and/or lymph nodes may offer a clinical benefit as a result of maintaining CAR-T cell regulation and CAR-T functional activation in an appropriate microenvironment. As for normal T cells (Zinkernagel RM, Ehl S, Aichele P, Oehen S, Kündig T, Hengartner H. Antigen localisation regulates immune responses in a dose- and time-dependent fashion: a geographical view of immune reactivity. Immunol Rev. 1997 Apr;156:199-209.), such lymphatic microenvironment is expected to extend CAR-T activation and thus CAR persistence, but also regulate an overshooting of an immune response that can result in CRS and ICANS. In addition, sequestration of CAR-T away from the periphery is expected to reduce adverse events associated peripheral tissue activation and damage. FTY720 has been shown to enhance persistence of allogeneic T-bodies (Marcus et al., Blood 2011;118(4):975-983). Moreover, promoting sequestration of CAR cell may reduce the risk of GVHD occurrence in allogeneic CAR cell therapy (Depil et al., Nature Reviews Drug Discovery 19, 185-199(2020)).

Without limiting the invention to any particular mechanism, the invention described here meets this need, as the described combination of composition comprising CAR cells in combination with S1P receptor agonists should provide a synergistic therapeutic effect, by preventing CAR cells from leaving lymphoid tissues, and/or by further activating the CAR cells in killing mode, and/or furthermore by directly killing cancer cells, and consequently, improving the anti-tumor efficacy and/or preventing the relapse of hematological malignancies patients to who has been given the combination of CAR cells and a S1P receptor agonist. Sequestration of CAR cells in lymphoid tissues by S1P receptor agonists has also the potential to limit CRS, and/or MAS, post CAR cell therapy or other adverse events such as ICANS, which is observed clinically in all of the autologous cell therapies.

Accordingly, the disclosure provides, at least in part, compositions and methods of treating disorders such as cancer (e.g. hematological cancers or other B-cell malignancies) using immune effector cells (e.g., T cells or Natural Killer cells (NK cells)) that express a Chimeric Antigen Receptor (CAR) molecule (e.g., a CAR-That binds to a B-cell antigen, e.g., CD19).

The methods include administering immune effector cells (e.g., T cells or NK cells) expressing a CAR (e.g., a B cell targeting CAR) in combination with a S1P receptor agonist.

In some embodiments, the combination maintains CAR-T functional activity, has better clinical effectiveness, and/or has lower toxicity (e.g., due to prevention of CRS) as compared to either therapy alone. In some embodiments, the subject is at risk of, or has, CRS and/or MAS ; or the subject has been identified as having or being at risk of developing CRS and/or MAS . In some embodiments, the combination improves or ameliorates the anti-tumor efficacy of the CAR cell therapy, in particular a CAR cell therapy for treating a hematological malignancy.

The disclosure further pertains to the use of engineered cells, e.g., immune effector cells (e.g., T cells or NK cells), to express a CAR molecule that binds to a cancer-related antigen (e.g., cancer-related antigen described herein, e.g., CD19), in combination with a S1P receptor agonist (e.g., mocravimod) to treat a hematological cancer associated to said expression of said cancer-related antigen.

Also provided herein are compositions and methods for preventing CRS and/or MAS

in a subject by using a combination of a S1P receptor agonist (e.g. mocravimod) with a CAR-expressing cell (e.g., a B cell targeting CAR-expressing cell, e.g., anti-CD19 CAR cell).

Also provided are compositions and methods for preventing CRS and/or MAS, in a subject by using a combination of a S1P receptor agonist with a CAR-expressing cell (e.g., B cell targeting CAR- expressing cell, e.g., a CD19 CAR-expressing cell), e.g., where the subject is at risk of, or has, CRS and/or MAS; or the subject has been identified as having or at risk of developing CRS and/or MAS. In an aspect, provided herein is a method of treating a subject, e.g., a human, having a disease associated with expression of an antigen, e.g., cancer-related antigen described herein. The method comprises administering to the subject an effective amount of a cell e.g., an immune effector cell (e.g., a T cell or NK cell) that expresses a CAR molecule that binds the cancer-related antigen (e.g., cancer-related antigen as described herein, e.g., CD19), in combination with a S1P receptor agonist, e.g., mocravimod.

In another aspect provided herein is a method of providing anti-tumor immunity to a subject, e.g., humans, having a disease associated with expression of an antigen, e.g., cancer-related antigen, e.g., cancer-related antigen as described herein. The method comprises administering to the subject an effective amount of a cell e.g., an immune effector cell (e.g., a T cell or NK cell) that expresses a CAR molecule that binds the antigen (e.g., cancer-related antigen described herein, e.g., CD19), in combination with a S1P receptor agonist e.g. mocravimod.

In another aspect, provided herein is a method of treatment and/or preventing cytokine release syndrome (CRS), e.g., CRS associated with a CAR-Therapy (e.g., a CAR-expressing cell described herein) and/or macrophage activation syndrome (MAS), in a subject in need thereof, comprising administering a S1P receptor agonist (e.g., mocravimod), in combination with the CAR cell therapy, to the subject, thereby treating and/or preventing CRS and/or MAS in the subject.

In another aspect, provided herein is a method of increasing the efficacy of a CAR-Therapy (e.g., a CAR-expressing cell described herein) in a subject in need thereof, comprising administering a S1P receptor agonist (e.g., mocravimod), in combination with the CAR cell therapy, to the subject, thereby activating the CAR-expressing cells in killing mode, and/or by directly killing cancer cells, therefore increasing the anti-tumor effect.

In embodiments, the subject is at risk of developing, has, or is diagnosed with CRS and/or MAS. In embodiments, the subject has been, is being, or will be administered a CAR cell therapy, e.g., a CAR-expressing cell described herein.

In embodiments, the method comprises selecting the subject for administration of the S1P receptor agonist. In embodiments, the subject is selected based on (i) his or her risk of developing CRS and/or MAS, (ii) his or her diagnosis of CRS and/or MAS, and/or (iii) whether he or she has been, is being, or will be administered a CAR cell therapy (e.g., a CAR cell therapy described herein, e.g., anti-CD19 CAR cell therapy).

In embodiments, the subject is selected for administration of the S1P receptor agonist if the subject is diagnosed with CRS and/or MAS, e.g., severe (Grade 3 or 4) or non-severe CRS, and/or MAS. In embodiments, the subject is selected for administration of the S1P receptor agonist if the subject is at risk of (e.g., identified as at risk of) developing CRS and/or MAS. In embodiments, the subject is selected for administration of the S1P receptor agonist if the subject has been, is being, or will be administered a CAR cell therapy (e.g., a CAR cell therapy described herein, e.g., anti-CD19 CAR cell therapy).

Thus in one aspect, the disclosure provides a CAR cell composition for use in treating hematological malignancies in a subject in need thereof, wherein said CAR cell is an immune cell, preferably a T-cell, which expresses a chimeric antigen receptor molecule that binds a cancer-related antigen, and wherein a therapeutically effective amount of CAR cell composition is administered in combination with a therapeutically effective amount of an S1P receptor agonist.

In another aspect, the disclosure provides a CAR cell composition for use in treating metastatic tumors from solid tumor cancers, preferably lymphoid organs metastasis, more preferably lymph nodes metastasis, in a subject in need thereof, wherein said CAR cell is an immune cell, preferably a T-cell, which expresses a chimeric antigen receptor molecule that binds a cancer-related antigen, and wherein a therapeutically effective amount of CAR cell composition is administered in combination with a therapeutically effective amount of an S1P receptor agonist.

In another aspect, the therapeutically effective amount of an S1P receptor agonist is administrated before or at the time of the administration of the therapeutically effective amount of CAR cell composition.

In another aspect, CAR cells are treated *ex vivo* or *in vitro* with an effective amount of an S1P receptor agonist or its phosphate derivative before their administration.

Hence, the disclosure also provides activated CAR cell composition for use in treating hematological malignancies in a subject in need thereof, wherein said CAR cell is an immune cell, preferably a T-cell, which expresses a chimeric antigen receptor molecule that binds a cancer-related antigen, and wherein an therapeutically effective amount of activated CAR cell composition is administered to said subject, wherein said CAR cells have been treated *in vitro* or *ex vivo* with an effective amount of S1P receptor agonist or its phosphate derivative, for example mocravimod. In another aspect, the disclosure provides the use of a CAR cell composition for the manufacture of a medicament for treating hematological malignancies in a subject in need thereof, wherein said CAR cell is an immune cell, preferably a T-cell, which expresses a chimeric antigen receptor molecule that binds a cancer-related antigen, and wherein a therapeutically effective amount of CAR cell composition is administered in combination with a therapeutically effective amount of an S1P receptor agonist.

In another aspect, the disclosure provides methods for treating hematological malignancies, said method comprising administering a therapeutically effective amount of a CAR cell composition in combination with a therapeutically effective amount of a S1P receptor agonist, wherein said CAR cell is an immune cell, preferably an immune T-cell, which expresses a chimeric antigen receptor molecule that binds a cancer-related antigen (e.g. CD19).

As disclosed, the method may comprise the steps of:
1) collecting immune cells, for example immune T-cells, from a donor subject in need thereof by conducting leukapheresis,
2) ex vivo genetically modifying the donor subject's immune cells so that they express a chimeric antigen receptor (CAR) molecule that binds a cancer-related antigen (e.g. CD19), thereby obtaining CAR cells composition,
3) conditioning said recipient subject, for example by treating said recipient subject with an effective amount of a lymphodepleting chemotherapeutic agent or performing total body irradiation,
4) administering the composition comprising a therapeutically efficient amount of said CAR cells obtained at step 2) to said recipient subject, and
5) administering, for example before or after step 4), to the recipient subject an effective amount of a S1P receptor agonist, preferably mocravimod of formula II or a pharmaceutically acceptable salt thereof or a phosphate derivative thereof such as formula IIa or IIb, preferably before step 4),
6) optionally, administering to the recipient subject an effective amount of one or more immunosuppressants.

In another aspect, the disclosure provides a method of preventing or reducing cytokine release syndrome (CRS) and/or macrophage activation syndrome (MAS), with a CAR cell therapy (e.g; anti-CD19 CAR-T cell therapy) in a subject in need thereof, comprising administering a S1P receptor agonist (e.g. mocravimod) or a pharmaceutically acceptable salt thereof or a phosphate derivative thereof, in combination with the CAR cell therapy, to the subject, thereby preventing CRS and/or MAS in the subject.

In another aspect, the disclosure provides a S1P receptor modulator for use in preventing cytokine release syndrome, with a CAR cell therapy (e.g; anti-CD19 CAR-T cell therapy) in a subject in need thereof, comprising administering a S1P receptor agonist (e.g. mocravimod) or a pharmaceutically acceptable salt thereof or a phosphate derivative thereof, in combination with the CAR cell therapy, to the subject, thereby preventing CRS, and/or MAS, in the subject.

In another aspect, the disclosure provides a use of a S1P receptor modulator for the manufacture of a medicament for preventing cytokine release syndrome, and/or macrophage activation syndrome, with a CAR cell therapy (e.g; anti-CD19 CAR-T cell therapy) in a subject in need thereof, comprising administering a S1P receptor agonist (e.g. mocravimod) or a pharmaceutically acceptable salt thereof or a phosphate derivative thereof, in combination with the CAR cell therapy, to the subject, thereby preventing CRS, and/or macrophage activation syndrome, in the subject.

The disclosure is provided in various aspects as outlined in the claims.

### DETAILED DESCRIPTION

The disclosure includes methods of treating hematological malignancy in a subject in need thereof, comprising administering to said subject a therapeutically effective amount of CAR cell composition in combination with a therapeutically effective amount of an S1P receptor agonist.

The disclosure relates to a CAR cell composition for use in treating a hematological malignancy in a subject in need thereof, wherein said CAR cell is an immune cell, e.g. a T cell, which expresses a chimeric antigen receptor molecule that binds a cancer-related antigen (e.g. CD19), and wherein a therapeutically effective amount of CAR cell composition is administered in combination with a therapeutically effective amount of an S1P receptor agonist.

The disclosure also relates to the use of a CAR cell composition for the manufacture of a medicament for treating a hematological malignancy in a subject in need thereof, wherein a therapeutically effective amount of CAR cell composition is administered in combination with a therapeutically effective amount of an S1P receptor agonist.

The disclosure also relates to the use of a CAR cell composition in the preparation of a pharmaceutical composition for treating a hematological malignancy in a subject in need thereof, wherein a therapeutically effective amount of CAR cell composition is administered in combination with a therapeutically effective amount of an S1P receptor agonist.

### General Definitions

Unless defined otherwise, all technical and scientific terms used herein have the same meaning as commonly understood by one of ordinary skill in the art to which the invention pertains.

As used herein, a "modulator" is a compound which, when administered to a subject, provides the desired interaction with the target receptor, either by way of the compound acting directly on the receptor itself, or by way of a metabolite of the compound acting on the receptor. Upon administration to a subject, the S1P receptor modulator, preferably mocravimod (also referred as KRP203), interacts with the S1P receptor by either activating or inhibiting or downmodulating the receptor resulting in disrupted signal transduction.

As used herein, "S1P agonist" refers to a compound which initiates a physiological response when combined with the S1P receptor. Preferably the physiological response initiated is the agonist-induced internalization of the S1P receptor. The kinetics of agonist-induced internalization from cell membranes, and the recycling of the S1P receptors to the cell membrane after said compound shedding depends on the compound. Such S1P receptor agonists may also be referred as functional antagonists. The persistence of the internalization conditions the agonist's "functional antagonism" properties.

The term "pharmaceutically acceptable salts thereof" includes both acid and base addition salts. Non-limiting examples of pharmaceutically acceptable acid addition salts include chlorides, hydrochlorides, bromides, sulfates, nitrates, phosphates, sulfonates, methane sulfonates, formates, tartrates, maleates, citrates, benzoates, salicylates, and ascorbates. Non-limiting examples of pharmaceutically acceptable base addition salts include sodium, potassium, lithium, ammonium (substituted and unsubstituted), calcium, magnesium, iron, zinc, copper, manganese, and aluminum salts. Pharmaceutically acceptable salts may, for example, be obtained using standard procedures well known in the field of pharmaceuticals. For mocravimod, pharmaceutically acceptable salts would typically be acid-addition salts, since mocravimod is itself a base. Preferably, the pharmaceutically acceptable salts thereof is hydrochloride salt.

The term "phosphate derivatives thereof" includes phosphate esters such as of formula IIa or IIb.

The term "excipient", as used herein, refers to a non-active substance that is added alongside the drug substance, and is part of the formulation mixture. Pharmaceutically acceptable excipient are for example fillers, solvents, diluents, carriers, auxiliaries, distributing and sensing agents, delivery agents, such as preserving agents, disintegrants, moisteners, emulsifiers, suspending agents, thickeners, sweeteners, flavoring agents, aromatizing agents, antibacterial agents, fungicides, lubricants, and prolonged delivery controllers, antioxidants, glidants. The choice and suitable proportions of them are depended on the nature and way of administration and dosage.

An "average particle size" refers here to the D50 which means that 50% of the particles have size less than or equal to the indicated value. For instance, an average particle size less than or equal to 8 µm refers to a D50 of 8 µm, i.e. 50% of the particles have a particle size less than or equal to 8 µm. The term D90 means that 90% of the particles have a particle size less than or equal to the indicated value. For instance, a D90 less than or equal to 25 µm means that 90% of the particles have a particle size less than or equal to 25 µm. The D50 as well as the D90 are determined by laser light diffraction using the liquid route, e.g. on a BECKMAN-COULTER laser diffraction particle size analyzer LS 230, equipped with its small volume dispersion module (liquid route), following the technical manual and the manufacturer's instructions.

The term « Cytokine release syndrome » (CRS) refers to a significant complication associated with CAR cell therapy, which is an acute inflammatory process marked by a spectrum of clinical symptoms and substantial but transient elevations of serum cytokines. In most patients, CRS occurs 1-14 days after CAR-T cell infusion and rarely develops more than 17 days after infusion. The data suggest that CAR-T cell-associated CRS depends on T cell engagement with the target antigen followed by proliferation and functional response. Other factors influencing CRS may include disease type, nature and degree of lymphodepletion, and possibly CAR design. Because CRS is related to T cell engagement with a target antigen, it is not restricted to anti-CD19 therapies only. CRS was observed in four of seven (57%) evaluable pediatric or young adult patients with r/r ALL who received CD22-targeted CAR-T cells. In addition, CRS has been observed in patients with multiple myeloma (MM) who received CAR-T cells targeting CD19 or B cell maturation antigen (BCMA). The CRS grading scales developed by U.Penn is preferred (D. Porter et al., J Hematol Oncol. 2018; 11: 35).

The term "severe CRS" in contrast to the term "non severe CRS" refers to grade 3 or 4 CRS. Severe Grade 3 CRS is referring to hospitalization required for management of symptoms related to organ dysfunction, including grade 4 LFTs or grade 3 creatinine related to CRS and not attributable to any other conditions; this; includes hypotension treated with intravenous fluids (defined as multiple fluid boluses for blood pressure support) or low-dose vasopressors, coagulopathy requiring fresh frozen plasma or cryoprecipitate or fibrinogen concentrate, and hypoxia requiring supplemental oxygen (nasal cannula oxygen, high-flow oxygen, CPAP, or BiPAP). Severe Grade 4 CRS covers life-threatening complications such as hypotension requiring high-dose vasopressors, hypoxia requiring mechanical ventilation.

The term «macrophage activation syndrome » (MAS) refers to MAS-like symptoms observed in CAR cells treated patients, which can be life-threatening. MAS is sometimes synonymous with secondary hemophagocytic lymphohistiocytosis (HLH), is a term used by rheumatologists to describe a life-threatening complication of systemic inflammatory disorders, most commonly seen in systemic juvenile idiopathic arthritis (sJIA) and its adult equivalent, adult onset Still disease. Traditional MAS definitions are challenging to apply in patients receiving CAR-T cells, due to overlapping signs and symptoms with lymphodepletion and CRS. However, CRS symptoms develop first, characterized by fever and elevated C-reactive protein (CRP), and MAS (or MAS-like symptoms) then may develop after CRS, characterized by decrease in fibrinogen, rapid rise of ferritin and lactate dehydrogenase.

The term « Chimeric Antigen Receptor» or alternatively a « CAR » refers to artificial cell receptors for example T-bodies receptors, single-chain immunoreceptors, chimeric T-cell receptors, or chimeric immunoreceptors, for example, and encompass engineered receptors that graft an artificial specificity onto a particular immune effector cell. CARs may be employed to impart the specificity of a monoclonal antibody onto a T cell, thereby allowing a large number of specific T cells to be generated, for example, for use in adoptive cell therapy. In specific embodiments, CARs direct specificity of the cell to a tumor associated antigen, for example. In some embodiments, CARs comprise an intracellular activation domain (allowing the T cell to activate upon engagement of targeting moiety with target cell, such as a target tumor cell), a transmembrane domain, and an extracellular domain that may vary in length and comprises a disease- or disorder-associated, e.g., a tumor-antigen binding region. In particular aspects, CARs comprise fusions of single-chain variable fragments (scFv) derived from monoclonal antibodies, fused to CD3-zeta a transmembrane domain and endodomain. The specificity of other CAR designs may be derived from ligands of receptors (e.g., peptides) or from pattern-recognition receptors, such as Dectins. In certain cases, the spacing of the antigen-recognition domain can be modified to reduce activation-induced cell death. In certain cases, CARs comprise domains for additional co-stimulatory signaling, such as CD3ζ, FcR, CD27, CD28, CD137, DAP 10/12, and/or OX40, ICOS, TLRs (e.g., TLR2), etc. In some cases, molecules can be co-expressed with the CAR, including co-stimulatory molecules, reporter genes for imaging (e.g., for positron emission tomography), gene products that conditionally ablate the T cells upon addition of a pro-drug, homing receptors, chemokines, chemokine receptors, cytokines, and cytokine receptors. Furthermore, one skilled in the art will understand that a costimulatory domain need not be encoded solely by a full-length nucleotide sequence of a gene. It is readily apparent that the present invention includes, but is not limited to, the use of partial nucleotide sequences of more than one gene and that these nucleotide sequences are arranged in various combinations to elicit the desired immune response.

The term "anti-tumor effect" as used herein, refers to a biological effect which can be manifested by a decrease in tumor volume, a decrease in the number of tumor cells, a decrease in the number of metastases, an increase in life expectancy, or amelioration of various physiological symptoms associated with the cancerous condition.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen.

The terms "cancer-related antigen" or "tumor antigen" or "proliferative disorder antigen" or "antigen associated with a proliferative disorder" interchangeably refers to a molecule (typically protein, carbohydrate or lipid) that is expressed on the surface of a cancer cell, either entirely or as a fragment (e.g., MHC/peptide) and which is useful for targeting of a pharmacological agent to the cancer cell. In some embodiment the antigen tumor is a tumor specific antigen found on cancer cells only, not on healthy cells (TSA); or is a tumor associated antigens which have elevated levels on tumor cells, but are also expressed at lower levels on healthy cells (TAA). Interesting TSA are neo-antigens which are specific de novo cancer mutations. Other interesting TAA are intracellular targets that are exposed on the cellular surface by the MHC system (MHC-peptide antigens), such intracellular TAA associated with hematology cancers are, e.g., WT1, MAGE-4, Tyrosinase, PRAME, and Survivin.

In some embodiments, a tumor antigen is a marker expressed by both normal cells and cancer cells, e.g., a lineage marker, e.g., CD19 on B cells. In certain aspects, the tumor antigens of the present invention are derived from, cancers including but not limited to leukemia, lymphoma, diffuse Large B-Cell Lymphoma (DLBCL), chronic myelogenous leukemia (CML), acute myelogenous leukemia (AML), chronic lymphocytic leukemia (CLL), acute lymphocytic leukemia ALL, Hodgkin lymphoma, non-Hodgkin lymphoma, Mantle cell lymphoma (MCL), primary mediastinal large B-cell lymphoma (PMBCL) or multiple myeloma, preferably ALL, DLBCL, PMBCL and MCL.

In some embodiments, the tumor antigen is an antigen that is common to a specific proliferative disorder.

In some embodiments, a cancer-related antigen is a cell surface molecule that is overexpressed in a cancer cell in comparison to a normal cell, for instance, 1-fold over expression, 2-fold overexpression, 3-fold overexpression or more in comparison to a normal cell.

In some embodiments, a cancer-related antigen is a cell surface molecule that is inappropriately synthesized in the cancer cell, for instance, a molecule that contains deletions, additions or mutations in comparison to the molecule expressed on a normal cell. In some embodiments, a cancer-related antigen will be expressed exclusively on the cell surface of a cancer cell, entirely or as a fragment (e.g., MHC/peptide), and not synthesized or expressed on the surface of a normal cell. In some embodiments, the CARs of the present disclosure include TCR-like CARs comprising an antigen binding domain (e.g., antibody or antibody fragment) that binds to a MHC presented peptide. Normally, peptides derived from endogenous proteins fill the pockets of Major Histocompatibility Complex (MHC) class I molecules and are recognized by T cell receptors (TCRs) on CD8+ T lymphocytes. The MHC class I complexes are constitutively expressed by all nucleated cells. In cancer, virus-specific and/or tumor-specific peptide/MHC complexes represent a unique class of cell surface targets for immunotherapy.

Examples of preferred cancer-related antigens or combination of cancer-related antigens which can be targeted by CAR, typically via scFv or an engineered Tcell receptor, include CD4, CD5, CD7, CD19, CD19/CD20, CD19/CD22; CD20, CD22, CD20/CD22, CD30, CD33, CD37, CD38, CD39, CD70; CD123, CD44v6 (isoform variant 6), CD123/CLL1, BCMA CD38/BCMA, CD19/BCMA, CD56, CD138, LeY, ROR1, SLAMF7, nFLT3, LMP1, BCMA/TACI, CS1, GPRC5D, CS1/BCMA, NKG2D, CLL-1, GD2, MCSP, CD5, NKG2D ligands, MHC-peptide antigens. More preferably, said cancer-related antigens or combination of cancer-related antigens are selected from the group consisting of CD19, CD123, CD20, CD22, CD30, CD33, CD38, LeY, ROR1, CLL-1, BCMA, GD2, MCSP, CD5, NKG2D, MHC-peptide antigens or combination thereof, preferably CD19.

As used herein, a "CAR cell" refers to a cell expressing a CAR, and a CAR cell composition refers to a composition comprising CAR cells or a population of CAR cells. In some embodiments, cell expressing a CAR may be T cells, B cells, macrophage cells, dendritic cells, or NK cells. A therapy that comprises administering a therapeutically efficient amount of a CAR cell composition is referred herein a CAR cell therapy. The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules.

As used herein, "CAR cells activation" refers to the activation of CAR cells as detected by a significant increase of the cell or intracellular expression of one or more activation marker in CAR cells, which generally results to increased killing activity of the CAR cells. CAR-T cell activation may, for example, be detected by a significant increased expression of one or more of the following markers: surface PD1, intracellular Granzyme B and surface CD69.

The term « T cells » (or « T-cells » or "immune T-cells") refer to a type of lymphocyte that matures in the thymus. T cells play an important role in cell-mediated immunity and are distinguished from other lymphocytes, such as B cells, by the presence of a T-cell receptor on the cell surface. T-cells may either be isolated or obtained from a commercially available source. "T cell" includes all types of immune cells expressing CD3 including T-helper cells (CD4+ cells), cytotoxic T-cells (CD8+ cells), natural killer T-cells, T-regulatory cells (Treg) and gamma-delta T cells. A "cytotoxic cell" includes CD8+ T cells, natural-killer (NK) cells, and neutrophils, which cells are capable of mediating cytotoxicity responses. Non-limiting examples of commercially available T-cell lines include lines BCL2 (AAA) Jurkat (ATCC^{®} CRL-2902^{™}), BCL2 (S70A) Jurkat (ATCC^{®} CRL-2900^{™}), BCL2 (S87A) Jurkat (ATCC^{®} CRL-2901 ^{™}), BCL2 Jurkat (ATCC^{®} CRL-2899^{™}), Neo Jurkat (ATCC^{®} CRL-2898^{™}), TALL-104 cytotoxic human T cell line (ATCC # CRL-11386). Further examples include but are not limited to mature T-cell lines, e.g., such as Deglis, EBT-8, HPB-MLp-W, HUT 78, HUT 102, Karpas 384, Ki 225, My-La, Se-Ax, SKW-3, SMZ-1 and T34; and immature T- cell lines, e g., ALL-SIL, Be13, CCRF-CEM, CML-T1, DND-41, DU.528, EU-9, HD- Mar, HPB-ALL, H-SB2, HT-1, JK-T1, Jurkat, Karpas 45, KE-37, KOPT-K1, K-TI, L-KAW, Loucy, MAT, MOLT-1, MOLT 3, MOLT-4, MOLT 13, MOLT- 16, MT-1, MT-ALL,P12/Ichikawa, Peer, PER0117, PER-255, PF-382, PFI-285, RPMI-8402, ST-4, SUP-T1 to T14, TALL-1, T ALL-101, TALL-103/2, TALL-104, TALL-105, TALL-106, TALL-107,T ALL-197, TK-6, TLBR-1, -2, -3, and -4, CCRF-HSB-2 (CCL-120.1), J.RT3-T3.5 (ATCC TIB-153), J45.01 (ATCC CRL-1990), J.CaMI.6 (ATCC CRL-2063), RS4;11 (ATCC CRL- 1873), CCRF-CEM (ATCC CRM-CCL-119); and cutaneous T-cell lymphoma lines, e.g., HuT78 (ATCC CRM-TIB-161), MJ[G11] (ATCC CRL-8294), HuT102 (ATCC TIB-162). Null leukemia cell lines, including but not limited to REH, NALL-1, KM-3, L92-221, are a another commercially available source of immune cells, as are cell lines derived from other leukemias and lymphomas, such as K562 erythroleukemia, THP-1 monocytic leukemia,U937 lymphoma, HEL erythroleukemia, HL60 leukemia, HMC-1 leukemia, KG-1 leukemia, U266 myeloma. Non-limiting exemplary sources for such commercially available cell lines include the American Type Culture.

The term "T-reg" or "Tregs" refers to a T cell with immunosuppressive function. A T cell as used herein is a lymphocyte including any type of T cell, such as an alpha beta T cell (e.g. CD8 or CD4+), a gamma delta T cell, a memory T cell, a Treg cell etc. Suitably, immunosuppressive function may refer to the ability of the Treg to reduce or inhibit one or more of a number of physiological and cellular effects facilitated by the immune system in response to a stimulus such as a pathogen, an alloantigen, or an autoantigen. Examples of such effects include increased proliferation of conventional T cell (Tconv) and secretion of proinflammatory cytokines. Any such effects may be used as indicators of the strength of an immune response. A relatively weaker immune response by Tconv in the presence of Tregs would indicate an ability of the Treg to suppress immune responses. For example, a relative decrease in cytokine secretion would be indicative of a weaker immune response, and thus indicative of the ability of Tregs to suppress immune responses. Tregs can also suppress immune responses by modulating the expression of co-stimulatory molecules on antigen presenting cells (APCs), such as B cells, dendritic cells and macrophages. Expression levels of CD80 and CD86 can be used to assess suppression potency of activated Tregs in vitro after coculture.

The term "gamma delta T-cells" refers to a subset of T-cells that express a distinct T-cell receptor (TCR), namely γδTCR, on their surface, composed of one γ-chain and one δ-chain. The term "gamma-delta T-cells" specifically includes all subsets of gamma-delta T-cells, including, without limitation, Vδ1 and Vδ2, Vδ3 γδ T cells, as well as naive, effector memory, central memory, and terminally differentiated γδ T-cells. Compositions and methods for making and using engineered and non-engineered γδ T cells and/or sub-types thereof include, without limitation, those described in US 2016/0175358; WO 2017/197347; U.S. Pat. No. 9,499,788; US 2018/0169147; U.S. Pat. No. 9,907,820; US 2018/0125889 and US 2017/0196910, including the said compositions and methods for making and using engineered and non-engineered γδ T cells and/or sub-types thereof. The present application further contemplates T cells, or other engineered leukocytes or lymphocytes, that express one γ-chain or one δ-chain, optionally in combination with a second polypeptide to form a functional TCR. Such engineered leukocytes or lymphocytes, that express one γ-chain or one δ-chain may be used in the methods or present in the compositions described herein.

As used herein, the term "NK cell" also known as natural killer cell, refers to a type of lymphocyte that originates in the bone marrow and play a critical role in the innate immune system. NK cells provide rapid immune responses against viral-infected cells, tumor cells or other stressed cell, even in the absence of antibodies and major histocompatibility complex on the cell surfaces. NK cells may either be isolated or obtained from a commercially available source. Non-limiting examples of commercial NK cell lines include lines NK-92 (ATCC^{®} CRL-2407^{™}), NK-92MI (ATCC^{®} CRL-2408^{™}). Further examples include but are not limited to NK lines HANK1, KHYG-1, NKL, NK-YS, NOI-90, and YT. Non-limiting exemplary sources for such commercially available cell lines include the American Type Culture Collection, or ATCC, (www.atcc.org/) and the German Collection of Microorganisms and Cell Cultures (www.dsmz.de/).

As used herein the term "B cells" or " B lymphocyte or any term commonly used in the field refers to a type of lymphocyte that matures in the bone marrow to develop into a lymphocyte that functions in the humoral immunity component of the adaptive immune system. B cells produce antibody molecules that are membrane bound and do not secrete these antibodies. B cells, unlike the other two classes of lymphocytes, T cells and natural killer cells, express B cell receptors (BCRs) on their cell membrane. BCRs allow the B cell to bind to an antigen, against which it will initiate an antibody response. Naïve or memory B cell are activated by an antigen, depending on the antigen with the help or without the help of T cells, proliferate and differentiate into an antibody-secreting effector cell, known as a plasma blast or plasma cell. Additionally, B cells present antigens, are thus called professional antigen-presenting cells (APCs) and secrete cytokines, thereby modulate immune responses.. They have been uncovered as active participants in tumour-draining lymph nodes, tumour-associated tertiary lymphoid structures and tumor micro-environment to prompt anti-tumour response, although specific subsets are polarized with pro-tumoral effects. Their prevailing natures convey several advantages, making B cells attractive as a therapeutic cellular platform such as antigen-specific activation, in vivo persistence, memory pool formation and the potential to secrete proteins in large quantities. CAR-transduced leukemic B cell have been reported, e.g. clustered regularly interspaced short palindromic repeats (CRISPR) and CRISPR-associated protein 9 (Cas9) induced homolog-directed repair was successfully used to introduce CAR-expression cassettes into B cells. CARs equipped with the CD79β signalling domain, which is a component of B cell receptor (BCR) complex for activation, can be engineered in primary murine B cells to induce robust surface expression and antigen-recognition independent of endogenous BCR. B cells are therefore feasible carriers for CAR-based therapy by exploiting endogenous BCR signaling. CAR-B cells can be used to drive the local delivery of monoclonal antibodies at the tumour site by targeting a particular TAA. This introduces the possibility of CAR-B cells as safe and controllable vehicles for releasing efficacious therapeutic antibodies that convey severe toxicity in systemic administration. Alternatively, CAR-B cells can be a novel platform for autoimmune disease and prophylactic vaccines.

As used herein the term "macrophage cells" refers to innate myeloid cells are professional phagocytes capable of orchestrating homeostasis of the adaptive immune system. Given their high abundance in many solid tumors, tumor-associated macrophages (TAM) occupy a special niche in TME, and many mediators can tailor their phenotype [Immunosuppressive TAMs (M2) can dampen T cell response and facilitate tumor progression. In contrast, M1 polarization encompasses pro-inflammatory phenotype and harbors anti-tumor activity, thereby leading to great interest in engineering macrophages in cancer to assist immune surveillance. CAR endows macrophages with the specificity of response against tumor-associated antigens (TAAs) in parallel with enhanced effector functions against the tumor. For example, macrophages can be engineered with CD19-CAR incorporating cytosolic domains of Megf10 or FcRγ to mimic phagocytic signaling. Consequently, this triggered antigen specific phagocytosis and trogocytosis of lymphoma cells. CD3ζ-CAR macrophage also demonstrates active phagocytosis equivalent to FcRγ-CAR. As such, redirected antigen-specific phagocytosis bestows spatial control and precision on eliminating cancer cells and ultimately contributes to the therapeutic effect. Furthermore, macrophages may be transduced with conventional CAR via adenoviral vectors, and will polarize towards pro-inflammatory M1 phenotype and stimulated T cell responses, leading to marked tumor regression and prolonged survival.

As used herein the term "dendritic cells" (DC), refers to a heterogeneous subset of professional antigen-presenting cells that prime naïve T cells and reactivate memory responses. In cancer, DCs sense environmental cues in lymphoid organs or the tumor micro-environment and sensing of danger signals induces DC maturation leading to either immune tolerance or a tumor-specific response. Importantly, cytotoxic CD8+ T cells can be activated by DCs through cross-presentation of TAAs or neoantigens to promote a stronger anti-tumor response. These have key implications for cancer immunotherapy, and CAR becomes an emerging strategy to manipulate DCs for an effective response against the tumor.

The term "leukapheresis" as used herein refers to the art-recognized extracorporeal process by which the blood of a donor or patient is removed from the donor or patient and passed through an apparatus that separates out selected particular constituent(s) such as leukocytes and returns the remainder to the circulation of the donor or patient, e.g., by retransfusion.

The term "effective amount" or "therapeutically effective amount" refers to the amount of a composition that will elicit a biological or medical response of a cell, tissue, system, or subject that is being sought by the researcher, veterinarian, medical doctor or other clinician. For example, the composition comprises an effective amount of CAR cells, preferably anti-CD-19 CAR-T cells and/or mocravimod, that when administered to a subject, either as a single dose or as part of a series of doses, is effective to produce at least one therapeutic effect, e.g. sufficient to prevent development of, or alleviate to some extent, one or more of the signs or symptoms of the disorder or disease (e.g., hematological cancer) being treated. Effective amounts vary, as recognized by those skilled in the art, depending on, for example, route of administration, excipient usage, and co-usage with other active agents. In the case of treating a particular disease or condition, the desired therapeutic effect is inhibiting the progression of the disease. This may involve only slowing the progression of the disease temporarily, although more preferably, it involves halting the progression of the disease permanently. This can be monitored by routine methods or can be monitored according to diagnostic methods discussed herein. The desired response to treatment of the disease or condition also can be delaying the onset or even preventing the onset of the disease or condition. The precise amount of the composition comprising CAR cells to be administered can be determined by a physician with consideration of individual differences in age, weight, tumor size, extent of infection or metastasis, and condition of the patient (subject). In other embodiments, when referring to S1P receptor modulator, an effective amount of S1P receptor modulator (such as mocravimod) may refer to the amount sufficient to activate *in vivo, in vitro* or ex *vivo* the CAR cell compositions as defined herein, and/or to produce a synergistic therapeutic response when administered in combination with an effective amount of CAR cell composition in a subject in need thereof.

The term "about" has herein the meaning that the following value may vary for ± 20%, preferably ± 10%, more preferably ± 5%, even more preferably ± 2%, even more preferably ± 1%.

The terms "patient", "subject", "individual", and the like, are used interchangeably herein, and refer to a mammal, preferably a human. In some embodiments, the patient, subject or individual in need of treatment includes those who already have the disease, condition, or disorder, for example, hematological malignancies.

As used herein, the term "in combination" or "in combination therapy" means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered. In one embodiment, the CAR cells that express a CAR molecule that binds a cancer-related antigen is administered at a dose and/or dosing schedule described herein, and the S1P receptor modulator is administered at a dose and/or dosing schedule described herein. In some embodiments, "in combination with," is not intended to imply that the CAR cell therapy and the S1P receptor modulator (e.g., mocravimod, KRP203), must be administered at the same time and/or formulated for delivery together, although these methods of delivery are within the scope of this disclosure. The CAR cell therapy, can be administered concurrently with, prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or, preferably, subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after) a dose of the S1P receptor modulator, e.g., mocravimod. In certain embodiments, each agent will be administered at a dose and/or on a time schedule determined for that particular agent.

As used herein, unless specified otherwise, the term "treating" or "treatment", denotes reversing, alleviating, inhibiting the progress of, or preventing the disorder or condition to which such term applies, or reversing, alleviating, inhibiting the progress of, or preventing one or more symptoms of the disorder or condition to which such term applies.

As used herein, "Derived from" as that term is used herein, indicates a relationship between a first and a second molecule. It generally refers to structural similarity between the first molecule and a second molecule and does not connote or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connote or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

As used herein, the term "signaling domain" refers to the functional portion of a protein which acts by transmitting information within the cell to regulate cellular activity via defined signaling pathways by generating second messengers or functioning as effectors by responding to such messengers.

The term "genetically engineered" or "genetically modified" refers to a method of modifying the genome of a cell, including, but not limited to, deleting a coding or noncoding region or a portion thereof or inserting a coding region or a portion thereof. In some embodiments, the cell that is modified is an immune effector cell, for example lymphocyte, e.g., a T cell, a B cell or a NK cell, which can either be obtained from a patient or a donor. The cell can be modified to express an exogenous construct, such as, e.g., a chimeric antigen receptor (CAR), which is incorporated into the cell's genome.

As used herein, the term "CD19" refers to the Cluster of Differentiation 19 protein, which is an antigenic determinant expressed in all B lineage cells and detectable on leukemia precursor cells. The human and murine amino acid and nucleic acid sequences can be found in a public database, such as GenBank, UniProt and Swiss-Prot. For example, the amino acid sequence of human CD19 can be found as UniProt/Swiss-Prot Accession No. P15391 and the nucleotide sequence encoding of the human CD19 can be found at Accession No. NM_00I 178098. As used herein, "CD19" includes proteins comprising mutations, e.g., point mutations, fragments, insertions, deletions and splice variants of full length wild-type CD19. CD19 is expressed on most B lineage cancers, including, e.g., acute lymphoblastic leukemia, chronic lymphocyte leukemia and non-Hodgkin lymphoma. Other diseases associated with expression of CD19 are but not limited to hematological cancers, e.g leukemia, lymphoma, diffuse Large B-Cell Lymphoma (DLBCL), chronic myelogenous leukemia (CML), acute myelogenous leukemia (AML), chronic lymphocytic leukemia (CLL), acute lymphocytic leukemia ALL, Hodgkin lymphoma, non-Hodgkin lymphoma, Mantle cell lymphoma (MCL), primary mediastinal large B-cell lymphoma (PMBCL) or multiple myeloma, preferably ALL, DLBCL, PMBCL and MCL, more preferably DLBLC. It is also an early marker of B cell progenitors. See, e.g., Nicholson et al. Mol. Immun. 34 (16-17): 1157-1165 (1997). In one aspect the antigen-binding portion of the CART recognizes and binds an antigen within the extracellular domain of the CD 19 protein. In one aspect, the CD19 protein is expressed on a cancer cell.

As used herein, the term "CD20" refers to an antigenic determinant known to be detectable on B cells. Human CD20 is also called membrane-spanning 4-domains, subfamily A, member 1 (MS4A1). The human and murine amino acid and nucleic acid sequences can be found in a public database, such as GenBank, UniProt and Swiss-Prot. For example, the amino acid sequence of human CD20 can be found at Accession Nos. NP_690605.1 and NP_068769.2, and the nucleotide sequence encoding transcript variants 1 and 3 of the human CD20 can be found at Accession No. NM_152866.2 and NM_021950.3, respectively. In one aspect the antigen-binding portion of the CAR recognizes and binds an antigen within the extracellular domain of the CD20 protein. In one aspect, the CD20 protein is expressed on a cancer cell.

As used herein, the term "CD22," refers to an antigenic determinant known to be detectable on leukemia precursor cells. The human and murine amino acid and nucleic acid sequences can be found in a public database, such as GenBank, UniProt and Swiss-Prot. For example, the amino acid sequences of isoforms 1-5 human CD22 can be found at Accession Nos. NP 001762.2, NP 001172028.1, NP 001172029.1, NP 001172030.1, and NP 001265346.1, respectively, and the nucleotide sequence encoding variants 1-5 of the human CD22 can be found at Accession No. NM 001771.3, NM 001185099.1, NM 001185100.1, NM 001185101.1, and NM 001278417.1, respectively. In one aspect the antigen-binding portion of the CAR recognizes and binds an antigen within the extracellular domain of the CD22 protein. In one aspect, the CD22 protein is expressed on a cancer cell.

As used herein, the terms "alpha subunit of the IL-3 receptor," "IL3Ra," "CD123," "IL3Ra chain" and "IL3Ra subunit" refer interchangeably to an antigenic determinant known to be detectable on leukemia precursor cells. The human and murine amino acid and nucleic acid sequences can be found in a public database, such as GenBank, UniProt and Swiss-Prot. For example, the amino acid sequence of human IL3Ra can be found at Accession No. NP 002174 and the nucleotide sequence encoding of the human IL3Ra can be found at Accession No. NM 005191. In one aspect the antigen-binding portion of the CAR recognizes and binds an epitope within the extracellular domain of the CD123 protein. In one aspect, the CD123 protein is expressed on a cancer cell. As used herein, "CD123" includes proteins comprising mutations, e.g., point mutations, fragments, insertions, deletions and splice variants of full length wild-type CD123.

As used herein, the term "ROR1" refers to an antigenic determinant known to be detectable on leukemia precursor cells. The human and murine amino acid and nucleic acid sequences can be found in a public database, such as GenBank, UniProt and Swiss-Prot. For example, the amino acid sequences of isoforms 1 and 2 precursors of human ROR1 can be found at Accession Nos. NP_005003.2 and NP_001077061.1, respectively, and the mRNA sequences encoding them can be found at Accession Nos. NM_005012.3 and NM_001083592.1, respectively. In one aspect the antigen-binding portion of the CAR recognizes and binds an antigen within the extracellular domain of the ROR1 protein. In one aspect, the ROR1 protein is expressed on a cancer cell.

As used herein, the term "CD33" refers to the Cluster of Differentiation 33 protein, which is an antigenic determinant detectable on leukemia cells as well on normal precursor cells of the myeloid lineage. The human and murine amino acid and nucleic acid sequences can be found in a public database, such as GenBank, UniProt and Swiss-Prot. For example, the amino acid sequence of human CD33 can be found at UniProt/Swiss-Prot Accession No. P20138 and the nucleotide sequence encoding of the human CD33 can be found at Accession No. NM_001772.3. In one aspect the antigen-binding portion of the CAR recognizes and binds an epitope within the extracellular domain of the CD33 protein or fragments thereof. In one aspect, the CD33 protein is expressed on a cancer cell. As used herein, "CD33" includes proteins comprising mutations, e.g., point mutations, fragments, insertions, deletions and splice variants of full length wild-type CD33.

As used herein, the term "CD38" refers to the Cluster of Differentiation 38 protein, which is an antigenic determinant detectable on leukemia cells as well on normal precursor cells of the myeloid lineage. The human and murine amino acid and nucleic acid sequences can be found in a public database, such as GenBank, UniProt and Swiss-Prot. For example, the amino acid sequence of human CD38 can be found as UniProt/Swiss-Prot Accession No. P28907 and the nucleotide sequence encoding of the human CD38 can be found at Accession No. NM_001775. In one aspect the antigen-binding portion of the CAR recognizes and binds an epitope within the extracellular domain of the CD38 protein or fragments thereof. In one aspect, the CD38 protein is expressed on a cancer cell. As used herein, "CD38" includes proteins comprising mutations, e.g., point mutations, fragments, insertions, deletions and splice variants of full length wild-type CD38.

As used herein, the term "CLL-1" refers to human C-type lectin-like molecule-1 (CLL-1) which is a type II transmembrane glycoprotein, and its expression is restricted to myeloid cells and the majority of AML blasts. Moreover, CLL-1 is expressed in leukemia stem cells (LSCs), but absent in hematopoietic stem cells (HSCs), which may provide a potential therapeutic target for AML treatment (Wang, J., Chen, S., Xiao, W. et al. CAR-T cells targeting CLL-1 as an approach to treat acute myeloid leukemia. J Hematol Oncol 11, 7 (2018))

As used herein, the term "BCMA" refers to B-cell maturation antigen. BCMA (also known as TNFRSF17, BCM or CD269) is a member of the tumor necrosis factor receptor (TNFR) family and is predominantly expressed on terminally differentiated B cells, e.g., memory B cells, and plasma cells. Its ligand is called B-cell activator of the TNF family (BAFF) and a proliferation inducing ligand (APRIL). BCMA is involved in mediating the survival of plasma cells for maintaining long-term humoral immunity. The gene for BCMA is encoded on chromosome 16 and produces a primary mRNA transcript of 994 nucleotides in length (NCBI accession NM_001192.2) that encodes a protein of 184 amino acids (NP_001183.2). A second antisense transcript derived from the BCMA locus has been described, which may play a role in regulating BCMA expression. (Laabi Y. et al., Nucleic Acids Res., 1994, 22: 1147-1154).

The term "GD2" refers to disialoganglioside, a TAA found on NBLs, melanomas, and sarcomas.

The term "MCSP" refers to Melanoma Associated Chondroitin Sulfate Proteoglycan, another TAA found in melanomas.

The term "CD5" refers to the receptor CD5 expressed on the surface of T cells and in a subset of murine B cells known as B-1a. CD5 serves to mitigate activating signals from the BCR so that the B-1 cells can only be activated by very strong stimuli (such as bacterial proteins) and not by normal tissue proteins.

The term "NKG2D" refers to Natural Killer Group 2D (NKG2D) receptor which plays an important role in protecting the host from infections and cancer. By recognizing ligands induced on infected or tumor cells, NKG2D modulates lymphocyte activation and promotes immunity to eliminate ligand-expressing cells.

The term "scFv" refers to a fusion protein comprising at least one antibody fragment comprising a variable region of a light chain and at least one antibody fragment comprising a variable region of a heavy chain, wherein the light and heavy chain variable regions are contiguously linked via a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

As used herein, the term "relapse" or "relapsed" has its ordinary meaning in the art, and refer to the return of the hematological malignancy or the signs and symptoms of hematological malignancy after a period of complete remission (e.g, initial complete remission) due to treatment.

As used herein, the term "remission" has its ordinary meaning in the art, and refer to a decrease in or disappearance of signs and symptoms of cancer. In partial remission, some, but not all, signs and symptoms of cancer have disappeared. In complete remission (CR), all signs and symptoms of cancer have disappeared, although cancer still may be in the body.

As used herein, the terms "conditioning" indicates preparing a patient in need of an immune effector cell therapy for a suitable condition. Conditioning as used herein includes, but is not limited to, reducing the number of endogenous lymphocytes, removing a cytokine sink, increasing a serum level of one or more homeostatic cytokines or pro-inflammatory factors, enhancing an effector function of T cells administered after the conditioning, enhancing antigen presenting cell activation and/or availability, or any combination thereof prior to an immune effector cell therapy. In one embodiment, "conditioning" comprises lymphodepleting the patient.

The term "autologous" refers to any material derived from the same individual to whom it is later to be re-introduced into the individual. For example, the cell therapy method described herein involves collection of lymphocytes from a patient, which are then engineered to express, e.g., a CAR construct, and then administered back to the same patient.

The term "syngeneic" refers to any material derived from an individual which is genetically identical, for example the same individual or monozygotic twins, to whom it is later to be re-introduced into the individual.

The term "allogeneic" refers to any material derived from a different animal of the same species (e.g. human species) as the individual to whom the material is introduced. Two or more individuals are said to be allogeneic to one another when the genes at one or more loci are not identical. In some aspects, allogeneic material from individuals of the same species may be sufficiently unlike genetically to interact antigenically.

### Chimeric Antigen Receptor (CAR) cells

As used herein, a CAR cell composition is a compositing comprising cells or population of cells which have been genetically modified to express one or more chimeric antigen receptor molecules.

In specific embodiments, a CAR cell is a human immune effector cell or population of cells (e.g., a human T cell such as conventional T cell or regulatory T cell, or a human NK cell, e.g., a human T cell described herein or a human NK cell described herein). In one embodiment, the human T cell is a CD8+ T cell. In one embodiment, the cell is an autologous or syngeneic T cell. In one embodiment, the cell is an allogeneic T cell. It shall be understood that the compositions and methods disclosed herein reciting the term "cell" encompass compositions and methods comprising one or more cells, e.g., a population of cells.

Methods for producing CAR-T cells have been described for example in Feins et al. Am J Hematol. 2019;94:S3-S9. Methods for producing CAR NK cells have been described for example in Xie et al. EBiomedicine 59 (2020) 102975. Methods for producing CAR Treg cells have been described for example in Enrico Fritsche et al., Therapeutic Biomanufacturing, volume 38, issue 10, P1099-1112, October 2020.

The immune cells for producing the CAR cells for use according to the present may further comprise other genetic modifications (in addition to the one required for expression of the CAR molecules), in particular for allogeneic use, i.e. the CAR cells are made from immune cells of a donor subject which is different from the recipient subject.

In specific embodiments, the CAR construct includes a transmembrane domain selected from the group consisting of CD28, CD3, CD4, CD8α, and CD16.

In specific embodiments, the CAR construct comprises a cytoplasmic signaling domain selected from the group consisting of CD28, 4-1BB, ICOS, and CD27.

As used herein, the term CAR further encompasses chimeric antigen receptor molecules or combination of chimeric antigen receptor molecules which recognize a plurality of cancer-related antigen, typically two distinct cancer-related antigens (e.g. CD20/CD22), in particular to avoid tumor escape and/or mitigate toxicities. Such CAR recognizing more than one cancer-related antigen encompass without limitation dual CAR, tandem CAR, looped tandem CAR, combinatorial CAR, which are disclosed for example in Guedan et al 2019, Molecular Therapy: Methods & Clinical Development Vol. 12, pp 145-156.

CARs have been classified according to the modules that conform the cytoplasmic signaling domain. First generation CARs display a cytoplasmic signaling domain with one signaling module derived from the ζ-chain of the TCR/CD3 complex. Second and third generation are designed to include one or more than one costimulatory regions accompanying the CD3ζ chain, respectively. Although various costimulatory molecules including OX40, CD27 and ICOS, have been assessed in preclinical studies, CD28 and 4-1BB are the most commonly used in clinical trials.

First generation CAR-T cells exhibited cytotoxic activity in vitro, but they showed suboptimal persistence in vivo, limiting their therapeutic potential. In fact, second generation CARs have shown better T cell persistence after infusion compared to first-generation CARs. However, it has been reported that 4-1BB containing CARs exhibit a major persistence in vivo, in xenograft models as well as in patients, compared to those carrying CD28 domains. It was reported that CD28-based CAR-T cells have shown constitutive proliferation, effector memory differentiation and to be prone to exhaustion, while 4-1BB-based CAR-T cells exhibit central memory features with enhanced survival and exhaustion inhibition. Since costimulatory domains improved CAR-T cell function, survival and persistence, third generation CARs combining two co-stimulatory domains were generated. By linking the epitope specificity of a monoclonal antibody with the killing ability of a specific T cell, CARs bypass the requirement of antigen presentation by MHC molecules. Moreover, CAR-T cells can also recognize non-classical TCR targets such as lipids and carbohydrates, conferring CAR-T cells with the ability of recognizing a broader range of target antigens. Unlike TCR-transgenic T cells, CAR-T cells do not require antigen processing and are not HLA-restricted, which allows this therapy to reach a larger subset of patients. In addition, CAR-T cell infusion product is obtained from peripheral blood, so patients with no resectable tumors or with low T cell infiltration into the tumor (non-eligible for TIL therapy) can benefit from CAR-Therapy.

Hence, in one preferred embodiment, a CAR cell according to the present disclosure is a T cell expressing a CAR (i.e. a CAR-T cell), wherein said CAR comprises at least
(i) an extracellular domain comprising an antigen-binding domain, typically a single chain variable fragment with binding specificity to a cancer-related antigen (e.g; CD19),
(ii) a transmembrane domain, and
(iii) a cytoplasmic signaling domain, preferably comprising one signaling module derived from the ζ-chain of the TCR/CD3 complex, and optionally one or more costimulatory domains, typically CD28 or 4-1BB co-stimulatory domains.

Adoptive transfer of CAR-T cells has shown spectacular results in the treatment of B-cell derived malignancies. As a result, the U.S. Food and Drug Administration (FDA) has approved in 2017 the first two gene therapies, based on CAR-T cells, for B-cell malignancies. Kymriah (tisagenlecleucel) is a CD19-targeting second-generation CAR, containing a 4-1BB-derived costimulation domain, approved for the treatment of pediatric acute B-cell lymphoblastic leukemia. Yescarta (axicabtagene ciloleucel), also targeting CD19 antigen, is indicated for patients with diffuse large B-cell lymphoma. Unlike Kymriah, Yescarta contains a CD28-derived costimulatory module. In July 2020, the FDA approved Tecartus (brexucabtagene autoleucel) which also targets CD19 antigen for patients with mantle cell lymphoma that does not respond to other treatments or has recurred. In February 2021, the FDA approved Breyanzi (lisocabtagene maraleucel), which targets the CD19 antigen, for patients with relapsed or refractory large B-cell lymphoma. In March 2021, the FDA approved Abecma (idecabtagene vicleucel) as a first CAR-T cell targeting BCMA to treat adult patients with multiple myeloma.

The CAR cell as used in the present disclosure comprises a nucleic acid encoding a CAR comprising an extracellular domain comprising an antigen-binding domain, that specifically binds to a cancer-related antigen.

In specific embodiments, said cancer-related antigen is selected from the group consisting of cell surface markers of hematopoietic cells, preferably immune cells, such as B or T cells.

In one particular embodiment, the CAR cell for use in the present disclosure comprises a CAR with an extracellular domain comprising an antigen-binding domain that specifically binds to a cancer-related antigen selected from the group consisting of CD4, CD5, CD7, CD19, CD19/CD20, CD19/CD22; CD20, CD22, CD20/CD22, CD30, CD33, CD37, CD38, CD39, CD70; CD123, CD44v6 (isoform variant 6), CD123/CLL1, BCMA CD38/BCMA, CD19/BCMA, CD56, CD138, LeY, ROR1, SLAMF7, nFLT3, LMP1, BCMA/TACI, CS1, GPRC5D, CS1/BCMA, NKG2D, CLL-1 ligands. More preferably, said cancer-related antigens or combination of cancer-related antigens are selected from the group consisting of CD19, CD123, CD20, CD22, CD30, CD33, CD38, LeY, ROR1, CLL-1, GD2, MCSP, CD5, NKG2D, MHC-peptide antigens, BCMA, and combinations thereof, preferably CD19. In other specific embodiments, said cancer-related antigen is selected from cancer-related antigen typical of hematological malignancies, or cancer-related antigen of lymphoid organs metastasis, preferably lymph nodes metastasis, that may be from solid tumor cancers , in particular from breast cancers, typically HER2 or HER3. Said cancer-related antigen includes antigens that are expressed on the surface of the metastatic cancer or is expressed on the surface of the metastatic cancer in the context of a major histocompatibility complex molecule

In preferred embodiments, said preferred cancer-related antigens are selected from the group consisting of CD19, CD30, CD33, CD123, CD20, CD22, CD38, LeY, ROR1, CLL-1, GD2, MCSP, CD5, NKG2D, MHC-peptide antigens, BCMA, preferably CD19.

In specific embodiments, said CAR cells for use according to the present disclosure expresses a CAR that comprises, as an extracellular antigen-binding domain, a single chain variable fragment (scFv) that specifically binds to a cell surface marker selected from the group consisting of CD4, CD5, CD7, CD19, CD19/CD20, CD19/CD22; CD20, CD22, CD20/CD22, CD30, CD33, CD37, CD38, CD39, CD70; CD123, CD44v6 (isoform variant 6), CD123/CLL1, BCMA CD38/BCMA, CD19/BCMA, CD56, CD138, LeY, ROR1, SLAMF7, nFLT3, LMP1, BCMA/TACI, CS1, GPRC5D, CS1/BCMA, NKG2D ligands, and more preferably selected from the group consisting of CD19, CD30, CD33, CD123, CD20, CD22, CD38, LeY, ROR1, CLL-1, GD2, MCSP, CD5, NKG2D, MHC-peptide antigens, BCMA, preferably CD19.

Examples of CAR Constructs that binds a cancer-related antigen as described herein are described in WO2019/227003.

Other exemplary CAR constructs are those used in the following CAR-T commercial or development products: Kymriah (tisagenlecleucel), Yescarta (axicabtagene ciloleucel), Tecartus (brexucabtagene autoleucel), Breyanzi (lisocabtagene maraleucel), Abecma (idecabtagene vicleucel).

The composition comprising CAR cells as described herein is used in combination with a S1P receptor agonist.

### S1P receptor modulators

S1P receptors are divided into five subtype related G-coupled protein receptors (i.e., S1P₁, S1P₂, S1P₃, S1P₄ and S1P₅), which are expressed in a wide variety of tissues and exhibit different cell specificity.

In certain embodiments, a modulator of the S1P receptor for use according to the present methods of the disclosure is a compound which modulates one or more of the five S1P receptor types 1 to 5 (S1PR₁₋₅) by activating the receptor for signal transduction. Such compounds are also referred to herein as "S1P agonists".

In specific embodiments, said S1P receptor agonist for use in the treatment methods of the present disclosure is selected among KRP203 (mocravimod), FTY720 (fingolimod, Gilenya^{™}), BAF312 (siponimod, Mayzent^{®}), ozanimod (Zeposia^{®}), ponesimod, etrasimod, AKP-11, cenerimod, amiselimod, CBP-307, OPL-307, OPL-002, BMS-986166, SCD-044, BOS-173717, CP-1050. Preferably, said S1P receptor agonist for use in the treatment methods of the present disclosure is selected among KRP203 (mocravimod), FTY720 (fingolimod), and Mayzent^{®} (siponimod), most preferably mocravimod.

Examples of S1P agonists are KRP203 (mocravimod), a S1PR₁ selective agonist, or FTY720 (fingolimod), a multi-S1PR agonist of S1PR_{1,3-5} or siponimod, a S1PR₃ agonist, or any of their pharmaceutically acceptable salts or phosphate derivatives thereof. Preferably, in certain embodiments, said S1P agonist is selected among those S1P agonists selectively activating S1PR₁. In a preferred embodiment, the S1P receptor agonist for use according to the present disclosure is the compound of formula (I):

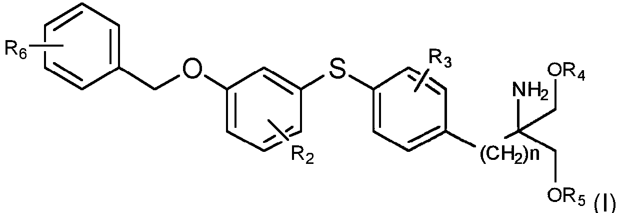

Wherein
R₂ is H, halogen, trihalomethyl, C₁₋₄alkoxy, C₁₋₇alkyl, phenethyl or benzyloxy;
R₃ is H, halogen, CF₃, OH, C₁₋₇alkyl, C₁₋₄alkoxy, benzyloxy, phenyl or C₁₋₄alkoxymethyl;
each of R₄ and R₅, independently is H or a residue of formula (a) 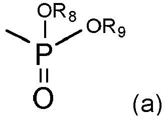
wherein each of R₈ and R₉, independently, is H or C₁₋₄alkyl optionally substituted by halogen;
and n is an integer from 1 to 4; and
R₆ is hydrogen, halogen, C₁₋₇alkyl, C₁₋₄alkoxy or trifluoromethyl.

In specific embodiments, said compoud of formula (I) is an S1P agonist, preferably an S1PR₁ selective agonist. Typically, in preferred embodiments, R₃ is chlorine. More preferably, R₂ is H, R₃ is chlorine and R₆ is hydrogen. For example, R₂ is H, R₃ is chlorine, R₆ is hydrogen, and each of R₃ and R₅, independently is H.

In a more preferred embodiment, the S1P receptor agonist for use according to the present disclosure, preferably an S1PR₁ selective agonist, is 2-amino-2- [4- (3-benzyloxyphenylthio) -2-chlorophenyl] ethyl-propane-1,3-diol, of formula (II) (also referred as mocravimod or KRP203): 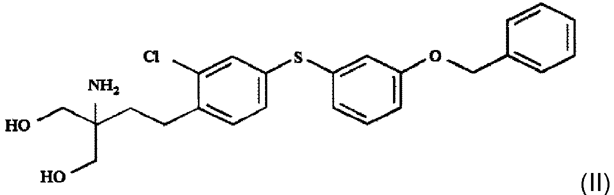 or pharmaceutically acceptable salts thereof.

Other S1P receptor agonist for use according to the present disclosure, preferably S1PR₁ selective agonist, includes the phosphate derivatives of the following formulae: 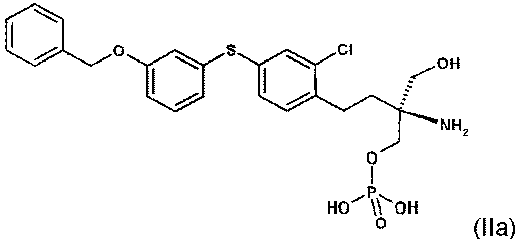 or

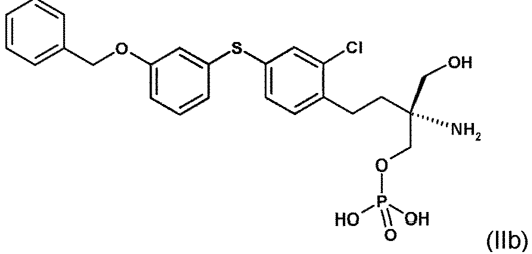

Said compounds and their synthesis methods are also disclosed in WO03/029205, WO2004/074297, WO2006/009092, WO2006/041019 and WO2014128611A1.

Mocravimod, or a pharmaceutically acceptable salt thereof, or a phosphate derivative thereof, is particularly preferred. Indeed, comparing pharmacodynamic effects of different S1P modulators, such as mocravimod, FTY720 and BAF312 established in healthy volunteers reveals differences in efficacy of lymphocyte sequestration. A measurable parameter that determines maintenance of the mode of action, i.e., sequestering of lymphocytes in secondary lymphoid organs and bone marrow, is reduction of peripheral lymphocyte counts. Recovery of absolute lymphocyte counts to 80% of normal counts after a single 1 mg dose of FTY720, multiple dose applications of BAF312 for 28 days, or a single 3 mg dose of KRP203 was reached after 8, 7 and more than 10 days, respectively. Thus, the lymphocyte recovery time for KRP203 is significantly longer than for BAF312 and FTY720.

### Particle size of the S1P receptor agonist

In the pharmaceutical industry, particle characterization of powder materials has become one of the crucial aspects in drug product development and quality control of solid oral dosage forms. The particle size distribution of the drug substance may have significant effects on final drug product performance (e.g., dissolution, bioavailability, content uniformity, stability, etc.). Furthermore, the particle size distributions of the drug substance can affect drug product manufacturability such as flowability, blend uniformity, compactibility, and have profound influence on almost every step of manufacturing processes for solid oral dosage forms, including pre-mixing/mixing, granulation, drying, milling, blending, coating, encapsulation, and compression. The particle size of the drug substance can therefore ultimately impact safety, efficacy, and quality of the drug product.

In an embodiment the S1P receptor agonist of the present disclosure has an average particle size (D50) of less than or equal to 8µm, preferably 6µm, more preferably 5µm. In another embodiment, the S1P receptor modulator of the present disclosure has a D90 of less than or equal to 25µm, preferably 22µm, more preferably 19µm. Indeed, it has been found by the inventors that an average particle size (D50) of greater than 8 µm, preferably greater than 6µm, more preferably greater than 5µm and/or a D90 greater than 25 µm, preferably greater than 22µm, more preferably greater than 19µm, impairs the dissolution of the drug by diminishing the surface contact with the solvent resulting in lowering bioavailability and in vivo performance.

### Pharmaceutical composition comprising the S1P receptor modulator

The present disclosure also relates to a pharmaceutical composition of said S1P receptor agonist, preferably mocravimod or a pharmaceutically acceptable salt thereof, or a phosphate derivative thereof, as described above, in particular for their use in the treatment methods as disclosed.

In an embodiment, the pharmaceutical composition of the present disclosure comprises the S1P receptor agonist, preferably mocravimod or a pharmaceutically acceptable salt thereof, or a phosphate derivative thereof, and one or more pharmaceutically acceptable excipients.

Any suitable excipients known to those of ordinary skill in the art for use in pharmaceutical compositions may be employed in the compositions described herein. The pharmaceutical composition may be administered in any manner appropriate to the disease or disorder to be treated as determined by persons of ordinary skill in the medical arts. An appropriate dose and a suitable duration and frequency of administration will be determined by such factors as discussed herein, including the condition of the patient, the type and severity of the patient's disease, the particular form of the active ingredient, and the method of administration. In general, an appropriate dose (or effective dose) and treatment regimen provides the pharmaceutical composition in an amount sufficient to provide a therapeutic effect, for example, an improved clinical outcome, such as more frequent complete or partial remissions, or longer disease-free and/or overall survival, or a lessening of symptom severity or other benefit as described in detail herein.

The pharmaceutical compositions described herein may be administered to a subject in need thereof by any of several routes that can effectively deliver an effective amount of the compound. The pharmaceutical composition may be administered orally, rectally, parenterally, intracisternally, intravaginally, intraperitoneally, topically, bucally, or as an oral or nasal spray. In a preferred embodiment, the pharmaceutical composition is suitable to be administered orally.

In another embodiment, the pharmaceutical composition may be a solid dosage form suitable for oral administration. Solid dosage forms for oral administration include capsules, tablets, pills, powders, and granules. In a preferred embodiment, the pharmaceutical composition is a capsule or a tablet. The capsule may be a soft or hard gelatin capsule, preferably a hard gelatin capsule. For example, the capsule is HGC Crushed or HPMC capsules Crushed.

In an embodiment, the release of the capsule or tablet content may be immediate or modified such as delayed, targeted or extended. In a preferred embodiment the solid dosage form is an immediate release dosage form.

In an embodiment, the pharmaceutical composition comprises the S1P receptor agonist, preferably mocravimod, and one or more pharmaceutically acceptable excipients, and particularly, at least one filler and mixtures thereof, a disintegrant, a lubricant and, a glidant.

### Fillers

Fillers, (also referred to as a diluents, dilutants or thinners) are substance which are added to the drug substance in order to make the latter suitable for oral administration (e.g., capsules, tablets). Fillers themselves should not produce any pharmacological effect on human being. Examples of fillers include mannitol, microcrystalline cellulose, lactose monohydrate, anhydrous lactose, corn starch, xylitol, sorbitol, sucrose, dicalcium phosphate, maltodextrin, and gelatin. The pharmaceutical composition of the present disclosure comprises at least one filler selected from mannitol, microcrystalline cellulose and mixtures thereof. In a preferred embodiment, the pharmaceutical composition of the present disclosure comprises a mixture of mannitol and microcrystalline cellulose.

### Disintegrants

Disintegrants are added to oral solid dosage forms to aid in their deaggregation. Disintegrants are formulated to cause a rapid break-up of solids dosage forms when they come into contact with moisture. Disintegration is typically viewed as the first step in the dissolution process. Examples of disintegrants include the modified starch such as sodium starch glycolate, sodium carboxymethyl starch, and pre-gelatinized starch, crosslinked polymers, such as crosslinked polyvinylpyrrolidone (crospovidone) or crosslinked sodium carboxymethyl cellulose (croscarmellose sodium), and calcium silicate. The pharmaceutical composition of the present disclosure comprises sodium starch glycolate as disintegrant.

### Lubricants

Lubricants are substances that we use in tablet and capsule formulations in order to reduce the friction. Lubricant can facilitate extrusion of tablets from matrix, thus preventing formation of scratches on their surfaces. By nature lubricants can be divided into two groups: a) fats and fat-like substances; b) powdery substance. Powdery substances are more applicable then the fat-like ones, because the latter impact on solubility and chemical stability of the tablets. Powdery lubricants are introduced by powdering of granulate. They provide constant-rate outflow of mass for tabletizing from hopper into matrix that guaranties accuracy and constancy of the drug substance dosage.

Examples of lubricants include magnesium stearate, hydrogenated castor oil, glyceryl behenate, calcium stearate, zinc stearate, mineral oil, silicone fluid, sodium lauryl sulfate, L-leucine, and sodium stearyl fumarate. The pharmaceutical composition of the present disclosure comprises magnesium stearate as lubricant.

### Glidants

Glidants are blended with the formulation to enhance the tablet-core blend-material flow property. During the early stage of compression, glidants are mixed within the particle arrangement of the tablet powder blend to improve flowability and uniformity within the die cavity of tablet presses. Glidants encourage the flow of tablet granulation by diminishing friction between particles. The effect of glidants on the flow of the granules depends on the size and shape of the particles of the granules and the glidants. Above a certain concentration, the glidant will in fact function to inhibit flowability. In tablet manufacture, glidants are usually added just prior to compression. Examples of glidants include colloidal silicon dioxide, starch, magnesium stearate and talc. The pharmaceutical composition of the present disclosure comprises colloidal silicon dioxide as lubricant.

The pharmaceutical composition of the present disclosure comprises the S1P receptor agonist, and at least one filler selected from mannitol, microcrystalline cellulose and mixtures thereof, sodium starch glycolate as disintegrant, magnesium stearate as lubricant and, colloidal silicon dioxide as glidant.

Any suitable excipients known to those of ordinary skill in the art in pharmaceutical compositions may be further employed in the compositions described herein.

In an embodiment the dosage strength of the S1P receptor agonist, preferably the hydrochloride salt of formula (I) or the phosphate derivatives of formula IIa or IIb, in the solid dosage form is between 0.05 mg to 15 mg/unit, preferably between 0.1mg to 10mg/unit, for example about 0.1mg/unit, or about 0.4mg/unit, or about 1 mg/unit, or about 10 mg/unit, more preferably about 1 mg/unit.

More specifically, the pharmaceutical composition of the present disclosure, in particular those comprising mocravimod or a pharmaceutically acceptable salt thereof, or a phosphate derivative thereof, at 1mg/unit, further comprises the following ingredients:
- mannitol, preferably at a content from 48 to 88 mg/unit, more preferably from 58 to 78mg/unit, even more preferably at a content about 68 mg/unit;
- microcrystalline cellulose, preferably at a content from 5 to 45 mg/unit, more preferably from 15 to 35 mg/unit, even more preferably at a content about 25 mg/unit;
- sodium starch glycolate, preferably at a content from 1 to 8 mg/unit, more preferably from 2 to 6 mg/unit, even more preferably at a content about 4 mg/unit;
- magnesium stearate, preferably at a content from 0.025 to 4 mg/unit, more preferably from 0.5 to 2 mg/unit, even more preferably at a content about 1 mg/unit; and
- colloidal silicon dioxide, preferably at a content from 0.125 to 2 mg/unit, more preferably from 0.25 to 1 mg/unit, even more preferably at a content about 0.5 mg/unit.

In another embodiment, the pharmaceutical composition of the present disclosure comprises the following ingredients:
- the S1P receptor agonist, preferably mocravimod, or a pharmaceutically acceptable salt thereof, or a phosphate derivative thereof, preferably at a content from 0.05% to 15%, more preferably from 0.1% to 10% mg/unit, even more preferably at a content about 0.1% or 0.4% or 1% or 10%;
- mannitol, preferably at a content from 48% to 88%, more preferably from 58% to 78%, even more preferably at a content about 68%;
- microcrystalline cellulose, preferably at a content from 5% to 45%, more preferably from 15% to 35%, even more preferably at a content about 25%;
- sodium starch glycolate, preferably at a content from 1% to 8%, more preferably from 2% to 6%, even more preferably at a content about 4%;
- magnesium stearate, preferably at a content from 0.025% to 4%, more preferably from 0.5% to 2%, even more preferably at a content about 1%;
- colloidal silicon dioxide, preferably at a content from 0.125 to 2 mg/unit, more preferably from 0.25 to 1 mg/unit, even more preferably at a content about 0.5 mg/unit,

Percentage being expressed mg/mg of the total composition in dry weight.

In an embodiment, the pharmaceutical composition of the disclosure is stable for at least 1 month at 50°C, preferably 2 months at 50°C.

In another embodiment, the pharmaceutical composition of the disclosure is stable at least 24 months at 5°C.

In another embodiment, the pharmaceutical composition of the disclosure is stable at least 24 months at 25°C/ 60% relative humidity.

As used herein, stability of a composition is measured according to the following method: high-pressure liquid chromatography (HPLC) which is widely known in the field. A composition is stable if the sum of impurities is less than or equal to 0.7% with a confidence interval of [98-102]%.

### Process of preparing the pharmaceutical composition

Another aspect of the present disclosure relates to the process of preparing the above-mentioned pharmaceutical composition, wherein it comprises the step of:
a. Blending the S1P receptor agonist or a pharmaceutically acceptable salt thereof or a phosphate derivative thereof with microcrystalline cellulose, colloidal silicon dioxide, preferably at 22rpm for 18 mins,
b. adding mannitol and blending the resulting mixture, preferably at 22rpm for 9 mins,
c. adding sodium starch glycolate and blending the resulting mixture, preferably at 22rpm for 5 mins,
d. adding magnesium stearate, blending the resulting pharmaceutical composition, preferably at 22rpm for 5 mins,
e. recovering the pharmaceutical composition of the present disclosure.

In a preferred embodiment, the process further comprises the step of:
f. filling the resulting pharmaceutical composition into capsules,
g. recovering the resulting capsules filled with the pharmaceutical composition.

### Patient population preferably targeted by the combination therapy

The treatment methods disclosed herein are suitable for patients having a hematological malignancy or lymphoid organs metastasis, preferably lymph nodes metastasis. Hematological malignancies are the types of cancer such as leukemia, lymphoma and malignant lymphoproliferative conditions that affect blood, bone marrow and the lymphatic system. Lymphoid organs metastasis, preferably lymph nodes metastasis, may be from solid tumor cancers, in particular breast cancer.

In one embodiment, the hematologic malignancy is leukemia. Leukemia can be classified as acute leukemia and chronic leukemia. Acute leukemia can be further classified as acute myeloid leukemia (AML) and acute lymphocytic leukemia (ALL). Chronic leukemia includes chronic myeloid leukemia (CML) and chronic lymphocytic leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

In other embodiment, the hematologic malignancy is lymphoma. Lymphoma is a group of blood cell tumors that develop from lymphocytes. Exemplary lymphomas include non-Hodgkin lymphoma and Hodgkin lymphoma.

Non-Hodgkin lymphoma (NHL) is a group of cancers of lymphocytes, formed from either B or T cells. NHLs occur at any age and are often characterized by lymph nodes that are larger than normal, weight loss, and fever. Different types of NHLs are categorized as aggressive (fast-growing) and indolent (slow-growing) types. B-cell non-Hodgkin lymphomas include Burkitt lymphoma, chronic lymphocytic leukemia/small lymphocytic lymphoma (CLL/SLL), diffuse large B-cell lymphoma (DLBCL), follicular lymphoma, immunoblastic large cell lymphoma, precursor B-lymphoblastic lymphoma, and mantle cell lymphoma. Examples of T-cell non-Hodgkin lymphomas include mycosis fungoides, anaplastic large cell lymphoma, and precursor T-lymphoblastic lymphoma. Lymphomas that occur after bone marrow or stem cell transplantation are typically B-cell non-Hodgkin lymphomas. See, e.g., Maloney. NEJM. 366.21(2012):2008-16. Diffuse large B-cell lymphoma (DLBCL) is a form of NHL that develops from B cells.

### Acute lymphocytic leukemia (ALL)

Acute lymphocytic leukemia (ALL) is a B-cell malignancy characterized by neoplastic cell proliferation and accumulation in bone morrow, blood, lymph nodes, and the spleen. ALL can arise in adults or in pediatric populations, can progress rapidly and can be fatal if left untreated. ALL includes relapsed and/or refractory ALL (r/r ALL). For relapsed and/or refractory ALL, treatment options include high-dose chemotherapy with subsequent allogeneic stem cell transplantation (SCT), standard chemo-immunotherapy, targeted treatment with small molecule pathway inhibitors, or supportive care with non-curative palliative goals. Allogeneic SCT is the only potentially curative option for r/r pediatric ALL, but outcomes are suboptimal. Among relapsed and/or refractory pediatric ALL patients who received allogeneic SCT in third or later remission, received allogeneic SCT with active disease or received allogeneic SCT after relapse from previous allogeneic SCT, the 1-year overall survival (OS) rates are 25 to 55% and 5-year OS rates are generally between 20 to 45%.

For ALL patients who are positive for the Philadelphia chromosome (Ph+), dasatinib (Sprycel) was approved in 2006 for the treatment of adult patients with resistance or intolerance to prior therapy. Ponatinib (Iclusig) was approved in 2013 for the treatment of adult patients with Ph+ ALL who are resistant to or intolerant of dasatinib. Blincyto (blinatumomab), a bispecific anti-CD3/CD19 monoclonal antibody, has been approved for the treatment of adults with Ph- relapsed or refractory B-precursor ALL. Despite the current treatment modalities, maintaining a remission in relapsed ALL patients is difficult, and the patients are being hospitalized for long periods of time with poor quality of life. The prognosis of patients with relapsed and/or refractory disease still remains poor.

### Diffuse Large B-Cell Lymphoma (DLBCL)

DLBCL is an aggressive lymphoma that can arise in lymph nodes or outside of the lymphatic system, e.g., in the gastrointestinal tract, testes, thyroid, skin, breast, bone, or brain. Three variants of cellular morphology are commonly observed in DLBCL: centroblastic, immunoblastic, and anaplastic. Centroblastic morphology is most common and has the appearance of medium-to-large-sized lymphocytes with minimal cytoplasm. There are several subtypes of DLBCL. Although most patients with DLBCL are adults, this disease sometimes occurs in children. Treatment for DLBCL includes chemotherapy (e.g., cyclophosphamide, doxorubicin, vincristine, prednisone, etoposide), antibodies (e.g., Rituxan), radiation, or autologous stem cell transplantation (ASCT). However, about half of patients relapsed and/or refractory to first-line therapy are not eligible for ASCT because of advanced age and/or comorbidities. Furthermore, among patients suitable for high-dose therapy and autologous stem cell transplantation (HD-ASCT), only about half have a response to salvage therapy that is sufficient to be able to proceed to HD-ASCT. In addition, of those proceeding to HD-ASCT, 60% of patients relapse after transplant. Clinical studies, palliative chemotherapy, and in rare cases a second HD-ASCT or allogeneic stem cell transplantation (AlloSCT) are some of the options available for these patients.

### Primary mediastinal large B-cell lymphoma (PMBCL)

Primary mediastinal large B cell lymphoma (PMBCL) has distinct clinical, pathological, and molecular characteristics compared to DLBCL. PMBCL is thought to arise from thymic (medullary) B cells and represents approximately 3% of patients diagnosed with DLBCL. PMBCL is typically identified in the younger adult population in the fourth decade of life with a slight female predominance. Gene expression profiling suggests deregulated pathways in PMBCL overlap with Hodgkin lymphoma. Initial therapy of PMBCL generally includes anthracycline-containing regimens with rituximab, such as infusional dose-adjusted etoposide, doxorubicin, and cyclophosphamide with vincristine, prednisone, and rituximab (DA-EPOCH-R), with or without involved field radiotherapy. Treatment options for relapsed/refractory PMBCL is similar to those in DLBCL. Patients with chemotherapy refractory disease have a similar or worse prognosis to those with refractory DLBCL.

### Mantle cell lymphoma (MCL)

MCL is an aggressive cancer that is poorly responsive to currently available therapies, i.e., essentially incurable. Mantle cell lymphoma arises in B cells, a type of white blood cells. In general, these patients have a poor prognosis, they repeated cycles of remission and relapse until the disease eventually becomes fatal. Mantle cell lymphoma has historically had one of the worst prognoses of any B-cell lymphoma, because the disease can be very aggressive, and chemotherapy does not provide a cure.

In one embodiment, the treatment methods disclosed herein are suitable for patients having metastatic tumors from solid tumor cancers. Solid tumors may be breast cancer, tripe negative breast cancer cells, ER-positive breast cancer, ER+ and ER-breast cancer, ER+ breast cancer, prostate cancer, pancreatic cancer, urothelial cancer, non-muscle invasive urothelial carcinoma cells, ovarian cancer, hypoxic ovarian cancer, bladder cancer, melanomas, lung cancers such as non-small cell lung cancer (NSCL), colorectal cancer, nephroblastoma such as Wilms tumor, thyroid carcinoma, thyroid cancer, hepatocellular carcinoma, cholangiocarcinoma neuroblastoma or glioblastoma multiforme. Indeed, these kinds of solid tumors may be drained in lymphoid organs, preferably lymph nodes, in such a way that metastasis can be found in such lymphoid organs, preferably lymph nodes. Therefore, the treatment methods disclosed herein are suitable for patients having lymphoid organs metastasis, preferably lymph nodes metastasis. Typically, the treatment methods as disclosed would help to eliminate metastasis found in lymphoid organs and especially in lymph nodes.In some embodiment the hematological malignancy is selected from the group consisting of leukemia, lymphoma, diffuse Large B-Cell Lymphoma (DLBCL), chronic myeloid leukemia (CML), acute myeloid leukemia (AML), chronic lymphocytic leukemia (CLL), acute lymphocytic leukemia (ALL), Hodgkin lymphoma (HL), non-Hodgkin lymphoma (NHL), Mantle cell lymphoma (MCL), primary mediastinal large B-cell lymphoma (PMBCL) or multiple myeloma.

Accordingly, the methods of the present disclosure are particularly suitable for subject with ALL, DLBCL, PMBCL and MCL.

In a preferred embodiment, the hematological malignancy is Diffuse Large B-Cell Lymphoma (DLBCL).

In specific embodiments, said subject is a subject eligible for CAR cell therapy for treating a hematological disorder. Typically, the subject has been, is being, or will be administered a CAR cell therapy, e.g., a CAR cell therapy described herein. In embodiments, the subject has been, is being, or will be administered an anti-CD19 CAR cell therapy.

In specific embodiments, said subject is at risk of developing cytokine release syndrome (CRS), and/or macrophage activation syndrome (MAS), and/or immune effector cell-associated neurotoxicity syndrome (ICANS), after CAR cell therapy, e.g. CAR-T cell therapy, e.g. anti-CD19 CAR-T cell therapy.

In specific embodiments, said subject has CRS, and/or MAS, and/or ICANS or is diagnosed with CRS, and/or MAS, and/or ICANS, after CAR cell therapy, e.g. CAR-T cell therapy, e.g. anti-CD19 CAR-T cell therapy.

In specific embodiments, said subject is at risk of GVHD after allogeneic CAR cell therapy.

### Combination therapy

The composition comprising CAR cells for use in combination therapy with a S1P receptor agonist as described herein are useful as a drug in methods for treating a hematological malignancy in a subject in need thereof, and more particularly in the population of patients and disease indications as defined above.

In one embodiment, said CAR cells, preferably CAR-T cells, and more preferably CAR-T cells targeting CD19 antigen, are administered at a dosage of between 0.1x10⁶ to 6x10⁸ CAR-positive viable cells/kg body weight. In another embodiment, said CAR cells, preferably CAR-T cells, and more preferably CAR-T cells targeting CD19 antigen, are administered 2 to 14 days after completion of the lymphodepleting chemotherapy.

In an embodiment the number of CAR cells in the peripheral blood of the subject is reduced between about 1% and about 40%, preferably between about 10% and about 30% as measured by flow cytometry after 7, preferably 14 days of S1P receptor modulator treatment, when compared to the number measured without S1P receptor agonist treatment.

In another embodiment, the amount of S1P receptor agonist can be administered per day at a fixed amount. Preferably said fixed daily dosage is 0,05 mg to 40 mg per day, preferably 0,1 mg to 35 mg, more preferably 0,5 mg to 30 mg, even more preferably 1 mg to 15 mg per day, even more preferably 1,5 mg to 7mg, even more preferably 2 mg to 5 mg, even more preferably about 3 mg per day or about 1 mg per day.

For example, the S1P receptor agonist can be mocravimod and said mocravimod may be administered at a daily dose of about 1 mg per day. Alternatively, mocravimod may be administered at a dose of about 3mg per day, preferably as three solid dosage forms of about 1mg or as one solid dosage form of about 3mg. Alternatively, mocravimod may be administered at a dose of about 2mg per day, preferably as two solid dosage forms of about 1mg or as one solid dosage form of about 2mg.

In some embodiment, said S1P receptor agonist is daily administered for at least 1, 2, or 3 months, or more, preferably from a starting day between 1 - 20 days prior said composition comprising CAR cells, more preferably 11 days before said T cell therapy. The composition comprising CAR cells that express a chimeric antigen receptor molecule that binds a cancer-related antigen, preferably CAR-T cells, and more preferably CAR-T cells targeting CD19 antigen (CD19 CAR-T cells), for use in combination therapy with a S1P receptor agonist, more preferably mocravimod, as described herein are useful in particular for preventing CAR cells from leaving the bone marrow and/or promoting CAR cell engraftment and persistence, thereby improving CAR cell therapy.

Detailed embodiments of such methods are disclosed hereafter.

In specific embodiments, the method of the present disclosure comprises the following steps:
1) collecting immune cells, for example immune T cells, from a donor subject in need thereof by conducting leukapheresis,
2) ex vivo genetically modifying the donor subject's immune cells so that they express a chimeric antigen receptor (CAR) molecule that binds a cancer-related antigen, thereby obtaining CAR cells composition,
3) conditioning said recipient subject, for example by treating said recipient subject with an effective amount of a lymphodepleting chemotherapeutic agent or performing body irradiation,
4) administering the composition comprising a therapeutically efficient amount of said CAR cells obtained at step 2) to said recipient subject, and
5) administering to the recipient subject an effective amount of a S1P receptor agonist, preferably mocravimod or a pharmaceutically acceptable salt thereof or a phosphate derivative thereof,
6) optionally, administering to the recipient subject an effective amount of one or more immunosuppressants.

Detailed embodiments of each step are described hereafter.

### Step (1) of collecting immune cells from the donor subject by leukapheresis

To perform the methods of treatment as disclosed herein, the immune cells from a donor subject are collected by leukapheresis wherein leukocytes are collected, enriched, or depleted ex vivo to select and/or isolate the cells of interest, e.g., T cells. The physician takes the subject's blood by using a catheter placed in the subject's vein. Some of the subject's white blood cells are separated from the patient's blood and the rest of the patient's blood is returned to the patient's vein. This procedure may take 3 to 6 hours and may need to be repeated in order to obtain enough white blood cells to perform step 2). Then the white blood cells are frozen and sent away to make genetic modifications.

In preferred embodiments, said donor subject is the same as the recipient subject in need of the treatment, i.e. said composition comprising CAR cells administered at step 4) comprises autologous or syngeneic CAR cells.

In other embodiments, said donor subject is not the same as the recipient subject in need of the treatment, i.e. said composition comprising CAR cells administered at step 4) comprises allogeneic CAR cells.

In preferred embodiments, said immune cells are immune effector cells selected from the group consisting of lymphocyte T cells or Natural Killer cells, more preferably lymphocyte T cells.

In certain aspects, the immune cells, for example T cells can be obtained from blood draws of from 10cc to 400cc. In certain aspects, the immune cells, for example T cells are obtained from blood draws of 20cc, 30cc, 40cc, 50cc, 60cc, 70cc, 80cc, 90cc, or 100cc.

### Step (2) of genetically modifying

In specific embodiments, the frozen white blood cells may be thawed, and the cells may be sorted to take preferentially the immune cells of interest, e.g. the T-lymphocytes or NK cells. These immune cells once isolated may be expanded by methods known in the art and treated such that one or more CAR constructs may be introduced.

The immune cells are genetically modified so that they express a CAR molecule that binds a cancer-related antigen as disclosed above. In a preferred embodiment, said cancer-related antigen is selected from the group consisting of CD19, CD30, CD33, CD123, CD20, CD22, CD38, LeY, ROR1, CLL-1, BCMA, GD2, MCSP, CD5, NKG2D, MHC-peptide antigens, more preferably CD19.

This procedure may take at least 2 weeks approximately.

### Step (3) of lymphodepleting

In order to prepare the patient's body to the CAR cell therapy, a conditioning of the patient or a conditioning regimen is performed unless the white blood cell count of the patient within one week prior to infusion is less than or equal to 1,000 cells/µL. The conditioning includes treatment of said patient with an effective amount of a lymphodepleting chemotherapeutic agent, before, after or concurrently with the administration of said therapy described herein.

Lymphodepleting chemotherapy as used herein is also referred to as lymphodepletion, a lymphodepleting therapy or a lymphodepleting regimen.

In some embodiments, a lymphodepleting chemotherapy is performed on a subject, prior to administering a CAR cell therapy described herein, e.g., CAR-T cell therapy, e.g., in combination with the S1P receptor agonist. In embodiments, the lymphodepletion comprises administering one or more (e.g., all) of melphalan, cytarabine, etoposide, busulfan, bendamustine, cyclophosphamide, fludarabine, dexamethasone and alemtuzumab, preferably cyclophosphamide, fludarabine, dexamethasone and alemtuzumab.

In some embodiments, a subject is administered lymphodepleting chemotherapy after administration of a CAR cell therapy described herein, e.g., CAR-T cell therapy, e.g., in combination with the S1P receptor agonist. For example, it is particularly the case when CAR cell therapy has to be performed one or several more times.

In a specific embodiment, when the delay between step 3) and step 4) is more than 4 weeks and the white blood cell count of the patient more than 1,000 cells/µL, then step 3) may be performed one more time prior performing step 4).

In an embodiment, said lymphodepleting chemotherapeutic agent is selected from the group consisting of cyclophosphamide, cytarabine, etoposide, bendamustine, busulfan, melphalan, fludarabine, and combinations thereof such as a combined administration of fludarabine/cyclophosphamide, cytarabine/etoposide.

In another aspect, said conditioning regimen of step 3) consists in :
- the administration of fludarabine and cyclophosphamide, or
- the administration of cytarabine and etoposide, or
- the administration of bendamustine.

In an embodiment, the fludarabine is daily administrated intravenously at a dosage of between 25 to 30 mg/m² for 3 or 4 days, and the cyclophosphamide is daily administrated intravenously at a dosage of between 250 to 500 mg/m² for 2 or 3 days starting with the first dose of fludarabine.

In another embodiment, the cytarabine is daily administrated intravenously at a dosage of 500 mg/m² for 2 days, and the etoposide is daily administrated intravenously at a dosage of 150 mg/m² for 3 days starting with the first dose of cytarabine.

In another embodiment, the bendamustine is daily administrated intravenously at a dosage of 90 mg/m² for 2 days.

### Step (4) of administering the composition comprising CAR cells

To perform the methods of treatment as disclosed herein, an effective amount of the composition comprising said CAR cells obtained at step (2) is administered to the subject in need of such treatment as described herein.

In specific embodiments, said CAR cells obtained at step (2), preferably CAR-T cells, may be activated *in vitro* or ex *vivo* by an effective amount of a S1P receptor modulator. Therefore, one aspect of the disclosure also pertains to methods of activating or pretreating CAR cells, preferably CAR-T cells, in particular for use in a method of treatment as described herein. In a specific method, a method comprises (i) pretreating CAR cells ex *vivo* or *in vitro* with an effective amount of a S1P receptor modulator for activating such CAR cells (preferably CAR-T cells) thereby obtaining activated CAR cells (preferably activated CAR-T cells), and then (ii) administering a therapeutically effective amount of obtained activated CAR cells to the subject in need of such treatment as described herein. In a specific embodiment of the latter method, no S1P receptor modulator is directly administered to the subject in combination with the activated CAR cell composition (except possibly trace amount of S1P receptor modulator which were used to activate (*in vitro* or ex *vivo*) the CAR cell composition.

The pharmaceutical compositions comprising said CAR cells may be administered in a manner appropriate to the disease to be treated (or prevented). The quantity and frequency of administration will be determined by such factors as the condition of the patient, and the type and severity of the patient's disease, although appropriate dosages may be determined by clinical trials.

In a preferred embodiment, the CAR cells of step 3) which are administered are CAR-T cell, e.g., anti-CD19 CAR-T cells.

The composition comprising said CAR cells may further comprise one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are in one aspect formulated for intravenous administration, preferably for infusion. The cells can be administered by using infusion techniques that are commonly known in immunotherapy (see, e.g., Rosenberg et al., New Eng. J. of Med. 319: 1676, 1988).

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

In an embodiment, the CAR cells of step 3), for example CAR-T cells, and in particular anti-CD19 CAR-T cells, are administered at a dosage of between 0.1x10⁶ to 6x10⁸ , 0.5x10⁶to 5.0x10⁸, 1.0x10⁶to 4.0x10⁸, 2.0x10⁶to 3.0x10⁸, 3.0 x10⁶ to 2.0x10⁸, 3.0x10⁶ to 1.0x10⁸, 4.0x10⁶ to 9.0x10⁷, 5.0x10⁶ to 8.0x10⁷ , 6.0x10⁶ to 7.0x10⁷, 7.0x10⁶ to 6.0x10⁷ , 8.0x10⁶ to 5.0x10⁷, 9.0x10⁶ to 4.0x10⁷, 1.0x10⁷ to 3.0x10⁷ CAR-positive viable T cells/kg body weight. In a preferred embodiment, the dose of the CAR cells of step 3) (e.g. CAR-T cells, and in particular anti-CD19 CAR-T cells) is of 1.2x10⁸ to 6x10⁸ CAR-positive viable immune cells/kg body weight. In another embodiment, the dose of the CAR cells of step 3) is of 0.4x10⁸ to 2x10⁸ CAR-positive viable immune cells/kg body weight.

In another embodiment, the CAR cells of step 3), (e.g. CAR-T cells, and in particular anti-CD19 CAR-T cells) are administered 2 to 14 days or 3 to 14 days after completion of the lymphodepleting chemotherapy.

The administration of the composition comprising the CAR cells may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the composition comprising the CAR cells is administered to a patient by intradermal or subcutaneous injection. In a preferred aspect, the T cell compositions of the present invention are administered by i.v. injection. The composition of the invention may be injected directly into a lymph node, or site of infection.

In one embodiment, the CAR is introduced into immune cells according to step 2), and the subject receives an initial administration of the CAR cells, and, eventually, one or more subsequent administrations of the CAR cells, wherein the one or more subsequent administrations are administered less than 15 days, e.g., 14, 13, 12, 11, 10, 9, 8, 7, 6, 5, 4, 3, or 2 days after the previous administration.

In one embodiment, more than one administration of the CAR cells is administered to the subject per week, e.g., 2, 3, or 4 administrations of the CAR cells of the invention are administered per week. In one embodiment, the subject receives more than one administration of the CAR cells per week (e.g., 2, 3 or 4 administrations per week) (also referred to herein as a cycle), followed by a week of no CAR cells administrations, and then one or more additional administrations of the CAR cells (e.g., more than one administration of the CAR cells per week) are administered to the subject.

In another embodiment, the subject receives more than one cycle of CAR cells, and the time between each cycle is less than 10, 9, 8, 7, 6, 5, 4, or 3 days. In one embodiment, the CAR cells are administered every other day for 3 administrations per week. In one embodiment, the CAR cells of the invention are administered for at least two, three, four, five, six, seven, eight or more weeks.

A potential issue that can arise in patients being treated using CAR cells (particularly with murine scFv bearing CARs) is anaphylaxis after multiple treatments. Without being bound by this theory, it is believed that such an anaphylactic response might be caused by a patient developing humoral anti-CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen-day break in exposure to the antigen. If a patient is at high risk of generating an anti-CAR antibody response during the course of transient CAR therapy (such as those generated by RNA transductions), CAR-T cells infusion breaks may not last more than 10 to 14 days.

In certain aspects, it may be desired to administer CAR cells to a subject and then subsequently redraw blood (or have an apheresis performed), genetically modifying the immune cells collected according to step (1), and reinfusing the patient with these CAR cells. This process can be carried out multiple times every few weeks.

### Step (5) of administering the S1P receptor agonist

To perform the methods of treatment as disclosed herein, an effective amount of S1P receptor agonist as described herein and preferably mocravimod, is administered to the subject in need of such treatment.

In some embodiments, the amount of S1P receptor agonist, preferably mocravimod or a pharmaceutically acceptable salt thereof or a phosphate derivative thereof, administered per day is a fixed amount. In some embodiments, the fixed daily dosage is 0,05 mg to 40 mg per day, preferably 0,1 mg to 35 mg, more preferably 0,5 mg to 30 mg, even more preferably 1 mg to 15 mg per day, even more preferably 1,5 mg to 7mg, even more preferably 2 mg to 5 mg, even more preferably about 3 mg per day or about 1 mg per day.

For example, the S1P receptor agonist can be mocravimod and said mocravimod may be administered at a daily dose of about 1 mg per day. Alternatively, mocravimod may be administered at a dose of about 3mg per day, preferably as three solid dosage forms of about 1mg or as one solid dosage form of about 3mg. Alternatively, mocravimod may be administered at a dose of about 2mg per day, preferably as two solid dosage forms of about 1mg or as one solid dosage form of about 2mg.

As used herein, the amount of a pharmaceutically acceptable salts or a phosphate derivatives of mocravimod refers to the amount of mocravimod base.

The composition comprising CAR cells as described herein and/or the S1P receptor agonist can be administered simultaneously, in the same or in separate compositions, or sequentially.

For sequential administration, in one embodiment, the composition comprising CAR cells described herein can be administered first, and the S1P receptor agonist can be administered second. In another specific embodiment of sequential administration, the S1P receptor agonist administration (typically as a daily dosage) starts prior to the first administration of a CAR cell composition.

In an embodiment, the S1P receptor agonist is administered prior to administration of the composition comprising CAR cells. In embodiments, the S1P receptor modulator is administered at least 1 day, 2 days, 3 days, 4 days, 5 days, 6 days, 7 days, 8 days, 9 days, 10 days, 11 days, 12 days, 13 days, 14 days, 15 days, 20 days prior to start of the administration of the CAR cell composition, preferably 11 days prior to start of the administration of the CAR cell composition.

In some embodiments, the S1P receptor agonist is mocravimod, and said mocravimod is daily administered for 1, 2, 3 months, or more, preferably from a starting day between 1 - 20 days prior to first administration of said composition comprising CAR cells (the CAR cell therapy), more preferably 11 days before start of the CAR cell therapy.

The daily dosage of S1P receptor agonist may be administered as one dose per day or in multiple doses in a single day. In a preferred embodiment, the daily dosage is administered once a day. In some embodiments, the doses are administered several times daily, preferably 3 times daily. The minimum dose that is enough to provide effective therapy may be used in some embodiments.

In another embodiment, said S1P receptor agonist is mocravimod, and said mocravimod is administered at a daily dose of 3mg, e.g. three solid dosage forms a day of 1mg. Indeed, 3 solid dosage forms, such as capsules or tablets, of 1mg administered in a spaced-apart manner is easier to swallow than a single solid dosage form of 3 mg.

In some embodiments, CAR cell therapy is administered to a subject having the hematological malignancies as described herein. The CAR cell therapy may be continued (for instance, when there are still some cancer-related antigen-expressing cancer cells detectable in the subject) or may be discontinued (for instance, when a risk-benefit analysis favors discontinuing the therapy).

In the CAR cell therapy where more than one administration of the composition comprising CAR cells is desired, the S1P receptor agonist regimen is initiated or completed prior to administration of the composition comprising CAR cells.

In some embodiments, one or more immunosuppressants are further administered to the patient. One or more immunosuppressants include but are not limited to any one or all of the following agents: ciclosporin A, sirolimus, tacrolimus, methotrexate, and mycophenolate, preferably ciclosporin A, or a combination of ciclosporin A and methotrexate.

In a preferred embodiment the method of the present disclosure, said S1P receptor agonist is administered in an amount sufficient for preventing CAR cells (typically CAR-T cells) from leaving the bone marrow, and/or lymph nodes and/or promoting CAR cells (typically CAR-T cells) engraftment and persistence and/or increasing the efficacy of a CAR cell therapy activation (typically CAR-T cells). Indeed, increasing the efficacy of a CAR cell therapy means activating such CAR cells which allows to improve the capacity of CAR cells to kill tumor cells and thus to better treat the hematological malignancy in a subject in need thereof i.e. increasing the anti tumor effect.

In another embodiment, said S1P receptor agonist is administered in an amount sufficient for reducing the risk of cytokine release syndrome (CRS), in particular systemic cytokine release syndrome, and/or macrophage activation syndrome (MAS), and/or immune effector cell-associated neurotoxicity syndrome (ICANS), in a subject receiving CAR cells. Indeed, reducing the risk of cytokine release syndrome, and/or macrophage activation syndrome, and/or immune effector cell-associated neurotoxicity syndrome allows to improve patient's response to CAR cells treatment. CRS being a result of administered CAR cells, especially CAR-T cells, becoming extensively activated resulting in the release of massive amounts of cytokines such as IFN-γ, TNF-α, IL-2, IL-4, IL-6, IL-10 and IL-17A, preventing the release of cytokines, would help preventing CRS, and thus improving patient's response to CAR cells treatment. MAS being a severe hyperinflammatory syndrome characterized by CRS and combinations of elevated serum ferritin and hemophagocytosis, renal failure, liver enzymes, splenomegaly, pulmonary edema, and/or absence of NK cell activity, preventing the release of cytokines, serum ferritin, liver enzymes, would help preventing MAS, and thus improving patient's response to CAR cells treatment.

ICANS being characterized by elevated cerebrospinal fluid cytokine levels and blood-brain barrier disruption, preventing the release of cytokine in the cerebrospinal fluid would help preventing ICANS, and thus improving patient's response to CAR cells treatment.

In consequence, the treatment of hematological malignancies, especially diffuse Large B-Cell Lymphoma (DLBCL), in subjects in need thereof will be improved.

In an embodiment, said S1P receptor agonist is administered in an amount sufficient for reducing the risk of cytokine release syndrome, in particular systemic cytokine release syndrome, in a subject receiving allogeneic CAR cells.

In preferred embodiments, said S1P receptor agonist is administered in an amount sufficient for reducing the risk of cytokine release syndrome, in particular systemic cytokine release syndrome, in a subject receiving autologous or syngeneic CAR cells.

Hence the present disclosure also relates to S1P receptor agonist for use in preventing or reducing the risk of cytokine release syndrome (CRS), in particular systemic cytokine release syndrome, and/or macrophage activation syndrome (MAS), and/or immune effector cell-associated neurotoxicity syndrome (ICANS), in a subject receiving CAR cell therapy in need thereof, typically undergoing a CAR cell therapy as disclosed above.

In specific embodiments of the methods where the recipient is not the donor of the CAR cells, for example the CAR cells are allogeneic, or universal CAR cells, said S1P receptor agonist is administered in an amount sufficient for reducing the risk of GVHD.

### FIGURES

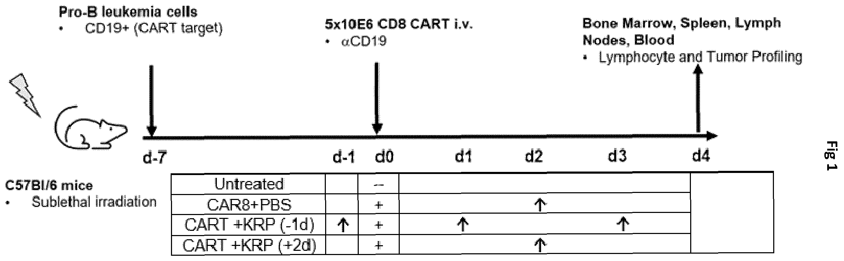

**Figure 1****:** Experimental scheme of example 1. Sublethal irradiation (4Gy) and 0.5x10⁶ leukemia injection IV in C57BI/6 mice at day -7. KRP203 3m/kg injection or PBS injection is represented by black arrows depending on the day of injection. The + and - indicates the administration or not of CAR T cells at day 0.

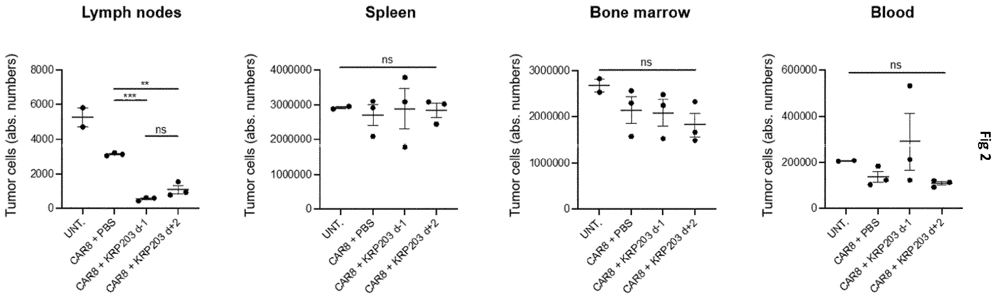

**Figure 2****:** Summary graphs showing the percentage of tumor cells recovered from lymph nodes, spleen, bone marrow and blood. Each dot represents one mouse. From left to right, light grey represents CAR8+PBS group, mid-grey represents CAR8+KRP203 D-1 group and dark grey represents CAR8+KRP203 D+2 group.

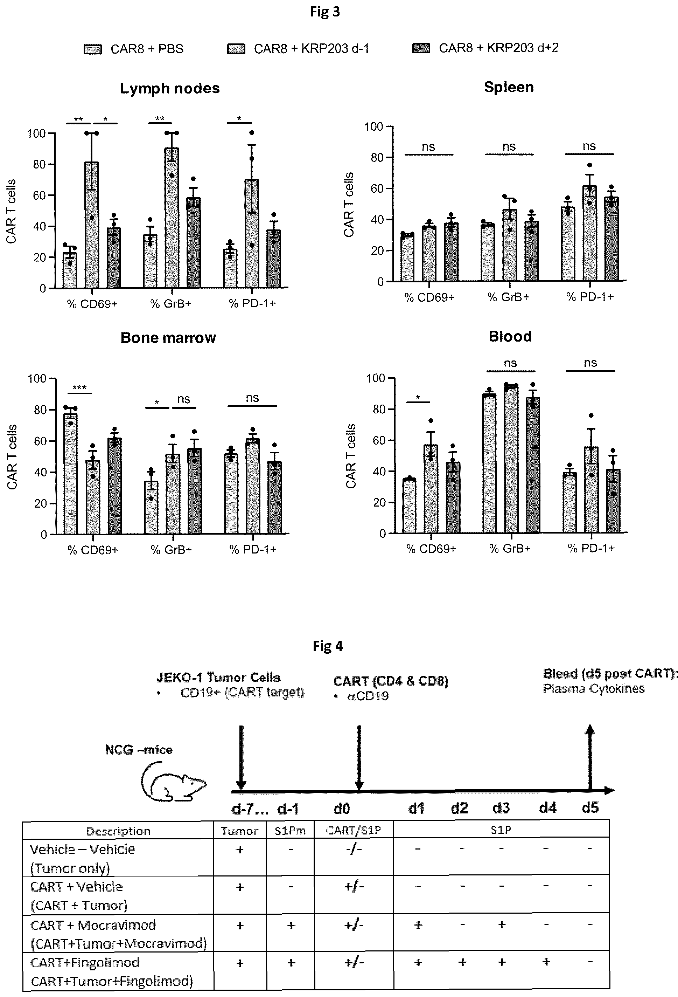

**Figure 3****:** Quantifications showing surface CD69, intracellular granzyme B and surface PD-1 expressions in CAR-T cells within lymph nodes, spleen, bone marrow and blood. Each dot represents one mouse.
**Figure 4****:** Experimental scheme of example 2.

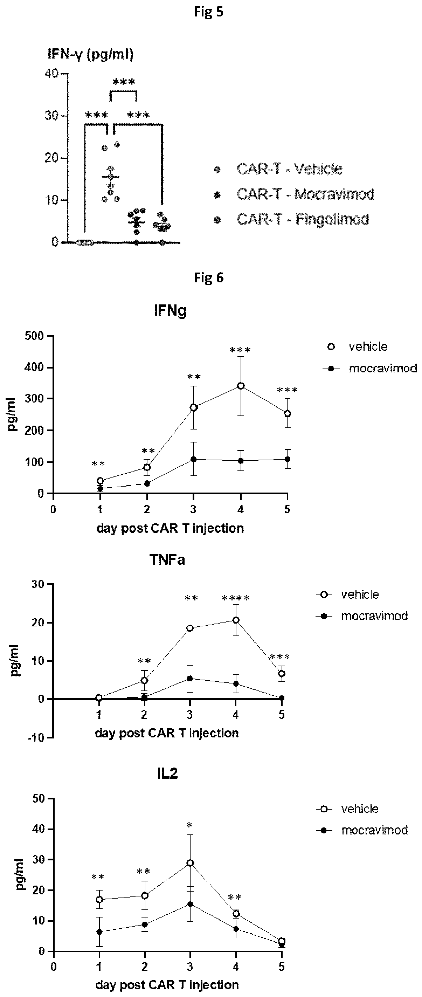

**Figure 5****:** Mocravimod (dark grey) and Fingolimod (mid-grey) decreased the level of IFN-γ induced by CAR-T cell injection. The level of (A) INF-γ was measured in plasma 5 days following the CAR-T injection. Individual value and means +/-SEM are shown. n=6-8 mice per group. A One-way ANOVA followed by a Dunnett post-test was used to compare all the treated groups between them. *** p < 0.001.
**Figure 6****:** Mocravimod (black) decreased the level of IFN-γ, TNF-α and IL-2 induced by CAR-T cell injection. The level of INF-g, TNF-a and IL-2 was measured in plasma 5 days following the CAR-T injection. Means +/-SD are shown. n=5 mice per group. An unpaired t test was used to compare vehicle and mocravimod groups on each time point. **p<0.005, ***p<0.001, ****p<0.0001

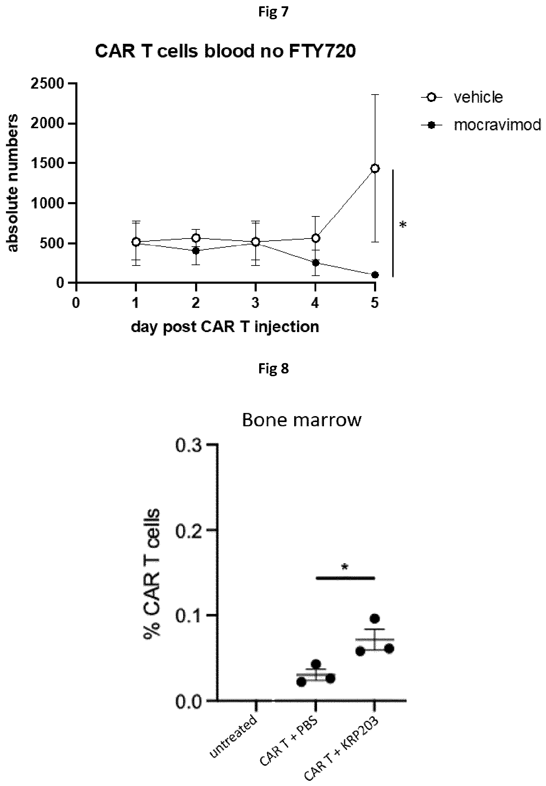

**Figure 7****:** Mocravimod decreased the absolute numbers of CAR-T cells in blood. The absolute numbers of CAR-T cells were measured in plasma on day 1 to 5 following the CAR-T injection. Means +/-SD are shown. n=5 mice per group. An unpaired t test was used to compare mocravimod to the vehicle group on day5. *p<0.05.
**Figure 8****:** Mocravimod increased the percentage of CAR-T cells in bone marrow compared to the untreated arm. CAR-T cells were measured in bone marrow 4 days following the CAR-T injection. Individual value and means +/-SEM are shown. n=3 mice per group. An unpaired t test was used to compare mocravimod to the control. *p < 0.05.

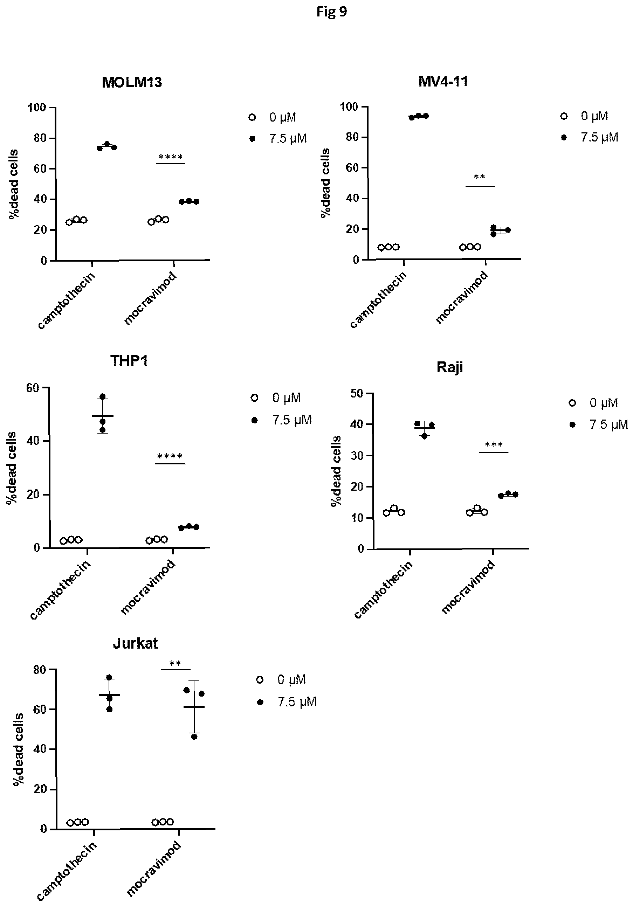

**Figure 9****:** Mocravimod kills tumor cells in vitro. An unpaired t test was used to compare no mocravimod to 7.5µM mocravimod. **p<0.005, ***p<0.001, ****p<0.0001.

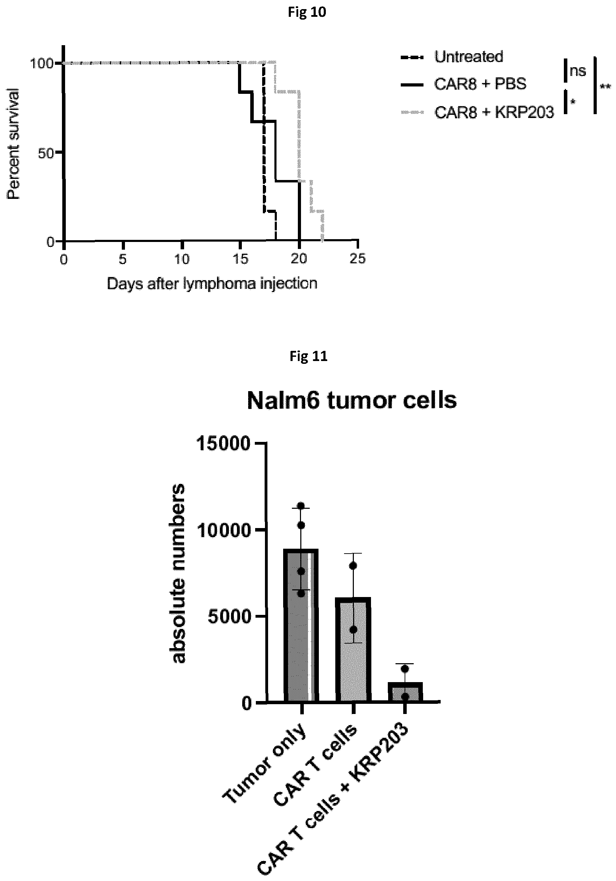

**Figure 10****:** Survival benefit of mocravimod in combination with CAR-T cells compared to CAR-T cells alone and untreated. *p<0.05, **p<0.01, ***p<0.001
**Figure 11****:** Mocravimod in combination with CAR-T cells increases tumor elimination.

### EXAMPLES

### Example 1: S1P modulators increase CAR-T activation

### Materials and methods

### Mice and cell line

6 to 8-week-old C57BL/6J male mice were obtained from Envigo. Ubi-GFP RAG1-/- OT-I TCR mice were bred and crossed in our animal facility under specific pathogen-free conditions. All experiments were approved by Institut Pasteur's Safety Committee in accordance with French and European guidelines (CETEA 2017-0038). Immortalized pro-B cells were generated by infecting bone marrow cells with a retrovirus encoding viral-Abelson kinase (v-abl). This tumor cell line was then retrovirally transduced to express a fluorescence resonance energy transfer (FRET)-based reporter for caspase 3 activity. Mice were examined every day and sacrificed in case of prostration, tousled hair, weakness or nodal tumor mass >1 cm. Cells were cultured in RPMI medium 1640-GlutaMAXTM supplemented with 10% heat-inactivated fetal bovine serum, 50 U.mL-1 penicillin, 50 µg.mL-1 streptomycin, 1 mM sodium pyruvate, 10 mM HEPES and 50 µM 2-mercaptoethanol, and maintained at 37°C and 5% CO2. Cells were routinely tested for the absence of Mycoplasma contamination (Venor-GeM Advance mycoplasma detection kit, Minerva Biolabs).

### CAR-T cell generation and adoptive transfer

The tCD34.2A.amCD19.CD28IEVζ retroviral vector encoding anti-CD19 CAR is composed of the anti-murine CD19 single-chain fragment variable domain derived from the 1D3 rat hybridoma, the transmembrane and intracellular domains of CD28, and the CD3z intracellular domain. The retroviral vector also encodes a truncated human CD34 molecule used for identification and purification of CAR-T cells. CD8+ T cells were isolated from lymph nodes of Ubi-GFP RAG1-/- OT-I TCR mice. T cells were activated in plates coated with 2.5 µg.ml-1 anti-CD3 mAb (clone 17.A2; BioLegend) in the presence of 2.5 µg.ml-1 soluble anti-CD28 mAb (clone 37.51; BioLegend) and 10 ng.ml-1 murine IL-12 (18523; Sigma-Aldrich). Two rounds of spin-infections were performed at 24 and 48 hours after T cell activation using retroviral particles supplemented with 8 µg.ml-1 polybrene (Merck). T cells were cultured for 4 additional days in the presence of 10 ng.ml-1 hIL-2 (202-IL; R&D Systems). CAR transduction efficacy was typically >80%; if lower, purification of transduced cells was performed using hCD34 positive selection kit (Miltenyi Biotec). B cell tumors were established by injecting 0.5x10⁶ transformed pro-B cells in mice following a sublethal irradiation (4 Gy), used as a conditioning regimen for CAR-T cell engraftment. Tumors develop primarily in the bone marrow and became detectable in the blood by day 7 at which time CAR-T cells (5x10⁶ cells) were injected intravenously (i.v.).

### KRP203 treatment

Mice received continuous KRP203 (3mg.kg-1 body weight) intraperitoneally every second day. KRP203 treatment was started either one day prior or 2 days after the injection of CAR-T cells. Control animals received PBS intraperitoneally. The experimental scheme is presented in Fig 1.

### Flow cytometry and antibodies

For ex vivo analysis, bone marrow cells were isolated by flushing femurs and tibias from tumor-bearing mice and subsequent filtering through 70 µm cell strainers. Single-cell suspensions from spleen and lymph nodes were prepared by filtering the cells through 70 µm cell strainers. Blood was collected by cardiac puncture after mouse sacrifice and red blood cells were removed using red blood cell lysis buffer (eBiosciences). Single-cell suspensions were Fc-blocked using anti-CD16/32 mAbs (clone 93; BioLegend) and normal murine serum 1%. Stainings were performed with the following mAbs: hCD34-Alexa Fluor 647 (clone 561; BioLegend), CD69-BUV737 (clone H1.2F3; BD Biosciences), CD8a-BUV395 (clone 53-6.7; BD Biosciences), CD19-APC-fire750 (clone 6D5; BioLegend) and PD-1-PE/Cy7 (clone 29F.1A12; BioLegend). Intracellular stainings were performed using the Cytofix/Cytoperm kit (BD Biosciences) according to the manufacturer's guidelines and PE-conjugated anti-Granzyme B mAb (clone QA18A28; BioLegend). Analyses were performed using a Cytoflex LX (Beckman Coulter) flow cytometer and analyzed with FlowJo v10.8.0 (BD).

### Statistical analysis

All statistical tests were performed using Prism v.9.2.0 (GraphPad). Data are expressed as mean ± SEM. One-way analysis of variance (ANOVA) and two-way ANOVA tests were used for multiple comparison correction. All statistical tests were two-tailed with a significance level of 0.05. ns, non-significant; *p<0.05; **p<0.01; ***p<0.001.

### Results

Leukemia tumor cells in distinct lymphoid organs have been assessed. Results are presented in Fig 2. The tumor burden, measured in absolute numbers of tumor cells, in lymph nodes was reduced in mice receiving CAR8 + PBS versus mice receiving only tumor cells (untreated group). It is demonstrated that when administering CAR-T cells and KRP203 at day -1 or d+2, the absolute tumor numbers in lymph nodes further decreases significantly in comparison to the control (CAR8+PBS) and the untreated arms. This means that when administering KRP203 in combination with CAR-T cells to leukemia mice in reduction of the absolute tumor numbers in lymph nodes can be achieved. The absolute tumor numbers in lymph nodes is further reduced when the administration of KRP203 is on day -1, day 1 and day 3 relative to administration of KRP203 on day 2 only.

CAR-T cell activation and elimination have been assessed. Results are presented in Fig 3. The results are consistent with Fig 2 with the reduction of the tumor burden in lymph nodes. Indeed, it is demonstrated that a higher proportion of CAR-T cells expressed the activation markers (CD69, GrB, PD-1), when combined with KRP203 treatment. In lymph nodes, when KRP203 is administered from day -1, the percentage of activation of CAR-T cells is significantly increased 2-3 times in comparison to the control. When administered day +2 there is also a trend of increased activation of CAR-T cells in comparison to the control but to a lesser extent compared to the day -1 administration. Increased activation markers (CD69, PD-1) indicates that CAR-T cells are more responsive to tumor cells and increased granzyme B (GrB) content that CAR-T cells have an enhanced capacity to kill cancer cells. Further, sequestering CAR-T cells with S1P modulators into lymph nodes may result in such CAR-T cells surviving longer.

Furthermore, survival of mice was investigated and how it is affected by administration of KRP203 in combination with CAR T cells. Mice treated with the combination of CAR T cells and KRP203 showed a statistically significant survival benefit compared to mice which were left untreated or received CAR T cells alone. (Figure 10)

### Example 2: Combination of S1P modulators with CAR-T cells prevents CRS

### Material & methods

### Animals

The study was carried out with female NOD/SCID/IL-2Rγnull immunodeficient mouse strain (NCG). All procedures described in this study were reviewed and approved by the local ethic committee (CELEAG). Mice were hosted by groups of 2/6 individuals in TCS BSL-2 animal facility. Each mouse was uniquely identified.

### Tumor cell engraftment

JEKO-1-luc-GFP tumor cells were expanded in vitro following ATCC's recommendation. 5x10⁶ tumor cells were grown in RPMI-1640 with 10% SVF and 1% Penicillin/Streptomycin, at 37°C in a water-saturated and sterile atmosphere with 5% CO². Following a viability check, tumor cells in logarithmic growth phase were injected in the selected animals.

JEKO-1-luc-GFP tumor cells were suspended at a concentration of 50x10⁶ cells/mL in PBS.

Tumor cells were injected intravenously in 24 immunodeficient mice with 100 µL of the cell suspension (5.106 JEKO-1 /mouse). Tumor engraftment was defined as D0.

### Randomization, groups and treatment

Mice were distributed across the 4 groups according to their body weight and were treated as follows: #1 Vehicle - Vehicle, #2 CAR-T - Vehicle, #3 CAR-T - Mocravimod and #4 CAR-T - Fingolimod.

Mocravimod and Fingolimod (FTY720) were administered at a dose of 3 mg/kg by intraperitoneal injection. Mocravimod and Vehicle groups (group #1, #2 and, #3) were treated every 2 days while Fingolimod group (#4) was treated daily starting on D6. CAR-T cells were administered once at D7 at a dose of 0.75x10⁶ cells per mouse (see table below).

| Group | Mice number | Treatment |
|---|---|---|
| 1 | 6 | *Vehicle (Sterile water +DMSO) at D6 + every 2 days by IP + Vehicle (PBS) once at D7 by IV |
| 2 | 6 | *Vehicle (Sterile water +DMSO) at D6 + every 2 days by IP + 0.75x10⁶ CAR-T cells once at D7 by IV |
| 3 | 6 | **Mocravimod 3 mg/kg at D6 + every 2 days by IP + 0.75x10⁶ CAR-T cells once at D7 by IV |
| 4 | 6 | ***Fingolimod 3 mg/kg at D6 + daily by IP + 0.75x10⁶ CAR-T cells once at D7 by IV |

| | | |
|---|---|---|
| *Vehicle solution injected for groups #1 and #2 mimicked the DMSO concentration used for the Mocravimod treated mice (#3). ** Mocravimod: Stock solution will be prepared at a concentration of 100 mg/ml with 100% DMSO and stored at -20°C. Every day of injections (every 2 days), one tube will be thawed and mixed by adding WATER to have a 0.75 mg/ml working solution. 100 µL of the working solution will be injected i.p. to the mice. *** One Fingolimod vial containing 25 mg will be resuspended with 2.5 ml of water. This will be the stock solution at 10 mg/ml. Stock solution will be aliquoted and stored at -20°C. Every day, one tube will be thawed and mixed by adding sterile water to have a 0.75 mg/ml working solution. 100 µL of the working solution will be injected i.p. to the mice. | | |

The experimental scheme is presented in Fig 4.

### Bleeding and organ collection

Blood was retro-orbitally collected at D12 for plasma isolation and CBA assay and at D25, the day of euthanasia for flow cytometry analysis.

Spleens and bone marrow were also harvested and mechanically dissociated in accordance with TransCure bioServices Standard Operating Procedures (SOP), and a red blood cell lysis was performed with RBC lysis buffer at RT for 1 min before flow cytometry analysis following internal TransCure bioServices protocols. Prior dissociation, a small part of the spleen was fixed in 4% PFA and paraffin embedded for further IHC analysis.

Obtained isolated cells were stained for immunophenotyping by flow cytometry, following internal TransCure bioServices protocols.

### Cytokine assay

Cytokine (TNF-α, INF-γ, IL-10, IL-6, IL-4, IL-2 and IL-17a) were analyzed on plasma samples (25 µL) by a customized Cytometric Bead Array (CBA) kit according to the manufacturer instructions (BD Biosciences) and TransCure standard procedures.

The level of several human cytokines (IFN-γ, TNF-α, IL-2, IL-4, IL-6, IL-10 and IL-17A) was measured on D12 (5 days following CAR-T cells injection) by cytometric bead array (CBA) assay (Figure 5). No or very low level of IL-2, IL4, IL-6, IL-10, IL-17A nor TNF-α were detected in blood of the mice regardless of the treated groups.

The cytokines profile analysis in blood 5 days after CAR-T cells administration showed that IFN-γ was only detected in the CAR-T cells treated mice. Intermediate levels of IFN-γ were found in the CAR-T - Mocravimod and CAR-T - Fingolimod treated groups (below 10 pg/mL) and significant higher level was found in the CAR-T - Vehicle group compared to the Vehicle - Vehicle group (Figure 5). In addition, the level of IFN-γ was significantly lower in the CAR-T - Mocravimod and in the CAR-T - Fingolimod groups compared to the CAR-T - Vehicle treated group.

### Conclusion

CAR-T cells induced a pro-inflammatory IFN-γ release which was significantly reduced upon Mocravimod or Fingolimod treatment. These results suggest that S1P modulators treatment would be useful prevent the cytokine release syndrome (CRS) induced by CAR-T cell therapy.

### Example 3: Combination of S1P modulators with CAR-T cells prevents CRS

### Material & methods

### Cytokine assay

To determine the kinetics of cytokine secretion and to evaluate the effectiveness of mocravimod in reducing the level of circulating cytokines when administered in combination with CAR T cells, NOD CRISPR Prkdc Il2r Gamma (NCG) mice were injected i.v. with 5x10⁶ JEKO-1-luc-GFP tumor cells. Treatment with either mocravimod or vehicle started on day 6 after tumor cell engraftment. Mocravimod (3 mg/kg) was administered every second day. 0.75x10⁶ CAR T cells were injected i.v. on day 7 after tumor cell engraftment. Mice were bled 24h, 48h, 72h, 96h and 120h post CAR T cell injection and plasma was collected. Interferon-g (IFN-g), Tumor necrosis factor-a (TNF-a) and Interleukin-2 (IL-2) were analyzed in plasma samples using the BD CBA Human Th1/Th2/Th17 cytokine assay. CAR T cells were phenotyped and enumerated in blood.

The cytokines profile analysis in blood 5 days after CAR-T cells administration showed that level of IFN-g, TNFa and IL-2 was significantly lower in the CAR-T - Mocravimod treated group (around 100 pg/mL, around 0 pg/mL and around 2 pg/mL respectively) compared to the CAR-T - Vehicle group (around 250pg/mL, around 6 pg/mL, around 3 pg/mL respectively) (Figure 6).

### CAR-T cells engraftment

The same protocol as example 1 has been conducted for the method of analyzing CAR-T cells in bone marrow and the same protocol as the cytokine assay has been conducted for the method of analyzing CAR-T cells in blood.

It is demonstrated that when starting administration of mocravimod on day -1 prior to CAR-T cell injection, the absolute numbers of CAR-T cells in blood significantly decrease in comparison to the control (vehicle) at day 5 (Figure 7). When comparing the combination of mocravimod with CAR-T cells to the control, the absolute number of CAR-T cells in blood at day 1 was the same (around 500). However, the absolute numbers of CAR-T cells in blood have the tendency to increase from day 3 to day 5 with the control (from around 500 to around 1500) whereas the absolute numbers of CAR-T cells in blood have the tendency to decrease with the combination of mocravimod with CAR-T cells (from around 500 to around 100).

These results are in agreement with the percentage of CAR-T cells in the bone marrow at day 4. When administering mocravimod with CAR-T cells, the percentage of CAR-T cells in bone marrow increased significantly in comparison to the control (CAR-T + PBS) (Figure 8). The results show, with the combination mocravimod with CAR-T cells, depletion of CAR-T cells in the blood and increase of CAR-T cells in the bone marrow which indicate sequestration of CAR T cells to lymphoid organs. Thus, the combination of mocravimod with CAR-T cells prevents cells from leaving the bone marrow, promotes CAR-T cells engraftment and increases the efficacy of a CAR-T cell therapy.

### Conclusions

CAR-T cells secreted pro-inflammatory cytokines IFN-γ, TNF-α and IL-2 which were significantly reduced upon Mocravimod treatment. These results confirm that S1P modulators treatment, and more preferably mocravimod, would be useful to prevent the cytokine release syndrome (CRS) induced by CAR-T cell therapy.

CAR-T cells have the tendency to leave the bone marrow and to be found in the blood. These results confirm that S1P modulators treatment would be useful for preventing CAR-T cells from leaving the bone marrow, promoting CAR cells engraftment and persistence and thus increasing the efficacy of a CAR-T cell therapy.

### Example 4: S1P modulators induces apoptosis

### Material & methods

To investigate cellular cytotoxicity (induction of cell death) of mocravimod on different blood cancer cell lines, cell lines were expanded and incubated for 2 and 24 hours in the presence of 7.5 µM mocravimod. The following cell lines were used: MOLM-13, THP-1, Kasumi-1, Jurkat, Raji, JEKO-1 and MV-4-11. 10⁵ tumor cells were seeded per well in a 96 well plate. 2 and 24hrs post incubation at 37°C, 5% CO₂ cells were stained with Annexin V and DAPI to evaluate cell death.

### Results

The percentage of dead cells of each tumor cell line MOLM-13 (AML), THP-1 (AML), Kasumi-1 (AML), Jurkat (ALL), Raji (B cell lymphoma), JEKO-1 (Mantle cell lymphoma) and MV-4-11 (AML) is higher when mocravimod is administered in the treated arm compared to the control untreated arm (Figure 9). Thus, mocravimod induces apoptosis of tumor cells in all MOLM-13, THP-1, Kasumi-1, Jurkat, Raji, JEKO-1 and MV-4-11 cell lines. Therefore the administration of mocravimod in combination with CAR-T cells allows to increase the efficacy of killing the hematologic malignancy.

### Example 5: Combination of mocravimod with CAR T cells increases tumor elimination

### Materials & methods

Scid-beige mice were injected with 0.5x10⁶ Nalm6-GFP⁺ tumor cells via tail vein (day 0). Tumor cells were previously expanded *in vitro.* While some mice remained untreated, some mice received 1x10⁷ CAR T cells and 1x10⁶ peripheral blood mononuclear cells (PBMCs) two weeks after tumor injection (day 14). Half of the mice who received CAR T cells were treated with mocravimod, while the other half received vehicle (DMSO). Mocravimod treatment started 3 days prior to CAR T cell injection at a dose of 3mg/kg and continued to be administered every second day until the end of the experiment. Tumor cells were analysed in bone marrow (BM) 4 days after CAR T cell administration (day 18).

### Results

Nalm6-GFP⁺ tumor cells were assessed in BM in all groups. Mice who received no CAR T cell treatment showed the highest tumor load (Figure 11). Tumor cells were efficiently reduced by CAR T cell administration. Furthermore, when KRP203 was administered in combination with CAR T cells, tumor cells were even more reduced. Therefore, the administration of mocravimod in combination with CAR-T cells allows to increase tumor elimination.

## Claims

1. A CAR cell composition for use in treating a hematological malignancy in a subject in need thereof, wherein said CAR cell is an immune cell, preferably an immune T-cell, which expresses a chimeric antigen receptor molecule that binds a cancer-related antigen, and wherein a therapeutically effective amount of said CAR cell composition is administered in combination with a therapeutically effective amount of an S1P receptor agonist.

2. The CAR cell composition for use according to claim 1, wherein said cancer-related antigen is selected from the group consisting of CD19, CD123, CD20, CD22, CD30, CD33, CD38, LeY, ROR1, CLL-1, BCMA, and combinations thereof, preferably CD19.

3. The CAR cell composition for use according to claim 1 or 2, wherein said S1P receptor agonist is selected among mocravimod, siponimod, fingolimod, ozanimod, ponesimod, etrasimod, AKP-11, cenerimod, amiselimod, CBP-307, OPL-307, OPL-002, BMS-986166, SCD-044, BOS-173717, CP-1050, preferably mocravimod.

4. The CAR cell composition for use according to claim 3, wherein the S1P receptor agonist is of the following formula (I) or (II) or (IIa) or (IIb): 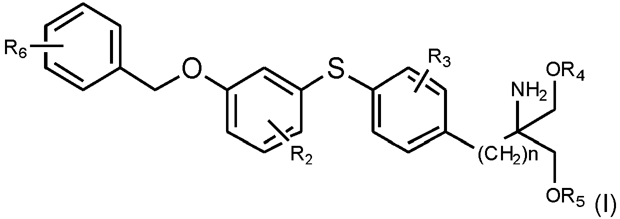 wherein
R₂ is H, halogen, trihalomethyl, C₁₋₄alkoxy, C₁₋₇alkyl, phenethyl or benzyloxy;
R₃ is H, halogen, CF₃, OH, C₁₋₇alkyl, C₁₋₄alkoxy, benzyloxy, phenyl or C₁₋₄alkoxymethyl;
each of R₄ and R₅, independently is H or a residue of formula (a) 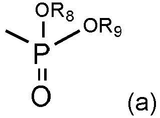
wherein each of R₅ and R₉, independently, is H or C₁₋₄alkyl optionally substituted by halogen;
and n is an integer from 1 to 4; and
R₆ is hydrogen, halogen, C₁₋₇alkyl, C₁₋₄alkoxy or trifluoromethyl,
Or, 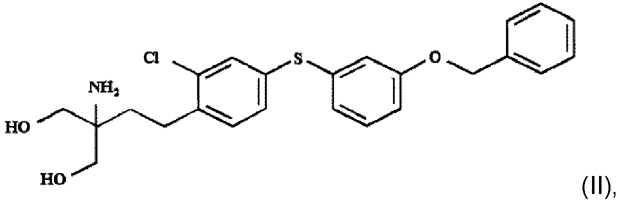
or pharmaceutically acceptable salts thereof
Or, 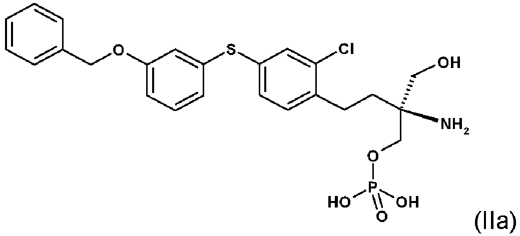 or

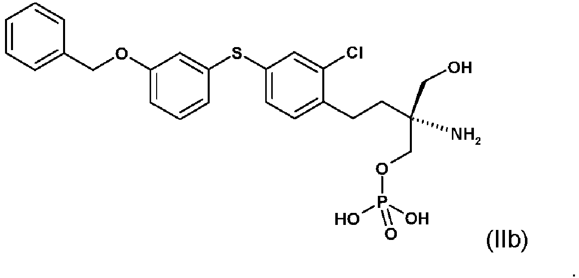

5. The CAR cell composition for use according to any one of claims 1-4, wherein the S1P receptor agonist is mocravimod, or a pharmaceutically acceptable salt thereof or a phosphate derivative thereof.

6. The CAR cell composition for use according to any one of Claims 1-5, wherein said hematological malignancy is leukemia and/or lymphoma, preferably selected from the group consisting of diffuse Large B-Cell Lymphoma (DLBCL), chronic myeloid leukemia (CML), acute myeloid leukemia (AML), chronic lymphocytic leukemia (CLL), acute lymphocytic leukemia (ALL), Hodgkin lymphoma, non-Hodgkin lymphoma, Mantle cell lymphoma (MCL), primary mediastinal large B-cell lymphoma (PMBCL) or multiple myeloma, more preferably ALL, DLBCL, PMBCL and MCL, even more preferably DLBCL.

7. The CAR cell composition for use according to any one of claims 1-6, wherein an efficient amount of CAR cells, preferably CAR-T cells, are administered at a dosage of between 0.1x10⁶ to 6x10⁸ CAR-positive viable immune cells/kg body weight.

8. The CAR cell composition for use according to any one of claims 1-7, wherein said CAR cells, preferably CAR-T cells, are administered 2 to 14 days after completion of the lymphodepleting chemotherapy.

9. The CAR cell composition for use according to any one of claims 1-8, wherein said S1P receptor agonist is administered per day at a dosage of between 0,05 mg to 40 mg, preferably 0,1 mg to 35 mg, more preferably 0,5 mg to 30 mg, even more preferably 1 mg to 15 mg, even more preferably 1,5 mg to 7mg, even more preferably 2 mg to 5 mg, even more preferably about 3 mg or about 1 mg .

10. The CAR cell composition for use according to any one of claims 1-9, wherein said S1P receptor agonist is daily administered for at least 1, 2, or 3 months, or more, preferably from a starting day between 1 - 20 days prior administering said composition comprising CAR cells, more preferably 11 days before CAR cell administration.

11. The CAR cell composition for use according to any one of claims 1-10, wherein said S1P receptor agonist is administered in an amount sufficient for preventing CAR cells from leaving the bone marrow and/or promoting CAR cells engraftment and persistence and/or increasing the efficacy of a CAR cell therapy.

12. The CAR cell composition for use according to any one of claims 1-11, wherein said S1P receptor agonist for use is administered in an amount sufficient for reducing the risk of cytokine release syndrome, in particular systemic cytokine release syndrome, in a subject receiving , preferably autologous or syngeneic CAR cells.

13. A S1P receptor agonist for use in preventing cytokine release syndrome (CRS) and/or macrophage activation syndrome (MAS), and/or immune effector cell-associated neurotoxicity syndrome (ICANS) with a CAR cell therapy (e.g; anti-CD19 therapy) in a subject in need thereof, comprising administering a S1P receptor agonist or a pharmaceutically acceptable salt thereof or a phosphate derivative thereof, in combination with the CAR cell therapy, to the subject, thereby preventing CRS, and/or MAS, and/or ICANS in the subject.

14. The S1P receptor modulator of claim 13, wherein said CAR cell therapy is a CAR-T cell therapy, preferably anti-CD19 CAR-T cell therapy.

15. The S1P receptor modulator of claim 13 or 14, wherein said S1P receptor modulator agonist is mocravimod, or a pharmaceutically acceptable salt thereof, or a phosphate derivative thereof.

## Patentansprüche

1. CAR-Zellzusammensetzung zur Verwendung zur Behandlung eines hämatologischen Malignoms bei einem Individuum, welches dessen bedarf, wobei die CAR-Zelle eine Immunzelle, bevorzugt eine Immun-T-Zelle, ist, welche ein chimäres Antigenrezeptormolekül exprimiert, welches ein Krebsbezogenes Antigen bindet, und wobei eine therapeutisch wirksame Menge der CAR-Zellzusammensetzung in Kombination mit einer therapeutisch wirksamen Menge eines S1P-Rezeptor-Agonisten verabreicht wird.

2. Die CAR-Zellzusammensetzung zur Verwendung nach Anspruch 1, wobei das Krebs-bezogene Antigen ausgewählt ist aus der Gruppe bestehend aus CD19, CD123, CD20, CD22, CD30, CD33, CD38, LeY, ROR1, CLL-1, BCMA, und Kombinationen davon, bevorzugt CD19.

3. CAR-Zellzusammensetzung zur Verwendung nach Anspruch 1 oder 2, wobei der S1P-Rezeptor-Agonist ausgewählt ist aus Mocravimod, Siponimod, Fingolimod, Ozanimod, Ponesimod, Etrasimod, AKP-11, Cenerimod, Amiselimod, CBP-307, OPL-307, OPL-002, BMS-986166, SCD-044, BOS-173717, CP-1050, bevorzugt Mocravimod.

4. CAR-Zellzusammensetzung zur Verwendung nach Anspruch 3, wobei der S1P-Rezeptor-Agonist der folgenden Formel (I) oder (II) oder (IIa) oder (IIb) entspricht: 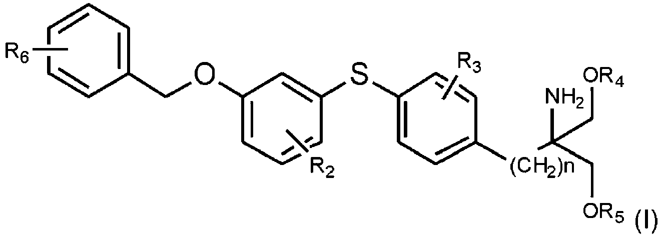 wobei
R₂ H, Halogen, Trihalomethyl, C₁₋₄-alkoxy, C₁₋₇-alkyl, Phenethyl oder Benzyloxy ist;
R₃ H, Halogen, CF₃, OH, C₁₋₇-alkyl, C₁₋₄-alkoxy, Benzyloxy, Phenyl oder C₁₋₄-alkoxymethyl ist;
jedes aus R₄ und R₅ unabhängig H oder ein Rest der Formel (a) ist 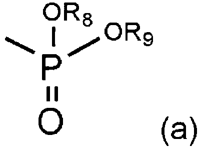
wobei jedes aus R₈ und R₉ unabhängig H oder C₁₋₄-alkyl, gegebenenfalls substituiert mit Halogen ist;
und n eine ganze Zahl von 1 bis 4 ist; und
R₆ Wasserstoff, Halogen, C₁₋₇-alkyl, C₁₋₄-alkoxy oder Trifluormethyl ist,
Oder 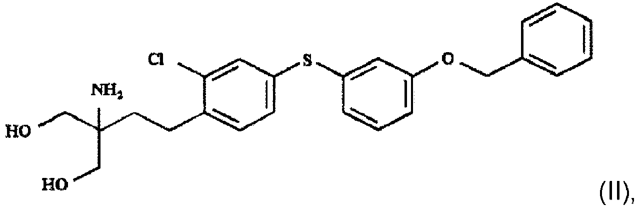
oder pharmazeutisch annehmbare Salze davon,
Oder 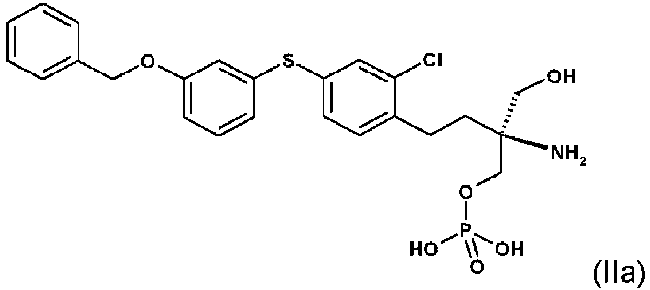 oder

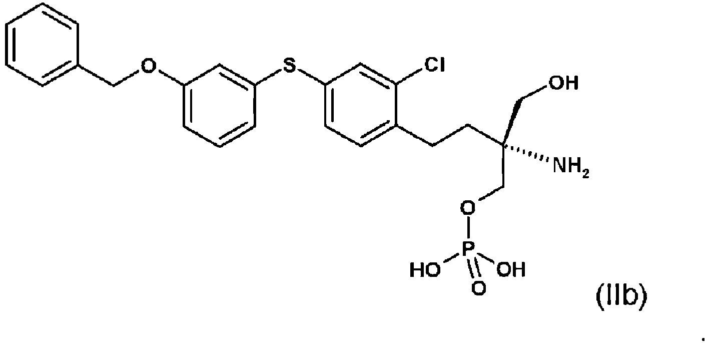

5. CAR-Zellzusammensetzung zur Verwendung nach einem der Ansprüche 1-4, wobei der S1P-Rezeptor-Agonist Mocravimod oder ein pharmazeutisch annehmbares Salz davon oder ein Phosphatderivat davon ist.

6. CAR-Zellzusammensetzung zur Verwendung nach einem der Ansprüche 1-5, wobei das hämatologische Malignom Leukämie und/oder Lymphom ist, bevorzugt ausgewählt aus der Gruppe bestehend aus diffusem großzelligem B-Zell-Lymphom (DLBCL), chronischer myeloischer Leukämie (CML), akuter myeloischer Leukämie (AML), chronischer lymphatischer Leukämie (CLL), akuter lymphatischer Leukämie (ALL), Hodgkin-Lymphom, Non-Hodgkin-Lymphom, Mantelzell-Lymphom (MCL), primärem mediastinalem großzelligem B-Zell-Lymphom (PMBCL) oder multiplem Myelom, stärker bevorzugt ALL, DLBCL, PMBCL und MCL, noch stärker bevorzugt DLBCL.

7. CAR-Zellzusammensetzung zur Verwendung nach einem der Ansprüche 1-6, wobei eine wirksame Menge an CAR-Zellen, bevorzugt CAR-T-Zellen, in einer Dosierung von zwischen 0,1 x 10⁶ und 6 x 10⁸ CAR-positiven lebensfähigen Immunzellen/kg Körpergewicht verabreicht wird.

8. CAR-Zellzusammensetzung zur Verwendung nach einem der Ansprüche 1-7, wobei die CAR-Zellen, bevorzugt CAR-T-Zellen, 2 bis 14 Tage nach Abschluss der lymphodepletierenden Chemotherapie verabreicht werden.

9. CAR-Zellzusammensetzung zur Verwendung nach einem der Ansprüche 1-8, wobei der S1P-Rezeptor-Agonist täglich in einer Dosierung von zwischen 0,05 mg bis 40 mg, bevorzugt 0,1 mg bis 35 mg, stärker bevorzugt 0,5 mg bis 30 mg, noch stärker bevorzugt 1 mg bis 15 mg, noch stärker bevorzugt 1,5 mg bis 7 mg, noch stärker bevorzugt 2 mg bis 5 mg, noch stärker bevorzugt etwa 3 mg oder etwa 1 mg verabreicht wird.

10. CAR-Zellzusammensetzung zur Verwendung nach einem der Ansprüche 1-9, wobei der S1P-Rezeptor-Agonist täglich für mindestens 1, 2 oder 3 Monate oder mehr verabreicht wird, bevorzugt ab einem Starttag zwischen 1-20 Tagen vor Verabreichen der Zusammensetzung umfassend CAR-Zellen, stärker bevorzugt 11 Tage vor CAR-Zellen-Verabreichung.

11. CAR-Zellzusammensetzung zur Verwendung nach einem der Ansprüche 1-10, wobei der S1P-Rezeptor-Agonist in einer Menge verabreicht wird, welche ausreichend ist zum Verhindern, dass CAR-Zellen das Knochenmark verlassen, und/oder zum Fördern des Anwachsens und Fortbestehens von CAR-Zellen und/oder zum Erhöhen der Wirksamkeit einer CAR-Zelltherapie.

12. CAR-Zellzusammensetzung zur Verwendung nach einem der Ansprüche 1-11, wobei der S1P-Rezeptor-Agonist zur Verwendung in einer Menge verabreicht wird, welche ausreichend ist zum Reduzieren des Risikos eines Zytokin-Freisetzungssyndroms, insbesondere systemischen Zytokin-Freisetzungssyndroms, in einem Individuum, welches bevorzugt autologe oder syngene CAR-Zellen erhält.

13. S1P-Rezeptor-Agonist zur Verwendung zum Verhindern von Zytokin-Freisetzungssyndrom (CRS) und/oder Makrophagenaktivierungssyndrom (MAS) und/oder Immuneffektorzell-assoziiertem Neurotoxizitätssyndrom (ICANS) bei einer CAR-Zelltherapie (z. B. Anti-CD19-Therapie) bei einem Individuum, welches dessen bedarf, umfassend Verabreichen eines S1P-Rezeptor-Agonisten oder eines pharmazeutisch annehmbaren Salzes davon oder eines Phosphatderivats davon in Kombination mit der CAR-Zelltherapie an das Individuum, wodurch CRS und/oder MAS und/oder ICANS bei dem Individuum verhindert werden.

14. S1P-Rezeptor-Modulator nach Anspruch 13, wobei die CAR-Zelltherapie eine CAR-T-Zelltherapie, bevorzugt Anti-CD19-CAR-T-Zelltherapie ist.

15. S1P-Rezeptor-Modulator nach Anspruch 13 oder 14, wobei der S1P-Rezeptor-Modulator-Agonist Mocravimod oder ein pharmazeutisch annehmbares Salz davon oder ein Phosphatderivat davon ist.

## Revendications

1. Composition de cellule CAR pour une utilisation dans le traitement d'une hémopathie maligne chez un sujet en ayant besoin, dans laquelle ladite cellule CAR est une cellule immunitaire, de préférence un lymphocyte T immunitaire, qui exprime une molécule de récepteur d'antigène chimérique qui se lie à un antigène lié au cancer, et dans laquelle une quantité thérapeutiquement efficace de ladite composition de cellule CAR est administrée en combinaison avec une quantité thérapeutiquement efficace d'un agoniste du récepteur S1P.

2. Composition de cellule CAR pour une utilisation selon la revendication 1, dans laquelle ledit antigène lié au cancer est sélectionné parmi le groupe consistant en CD19, CD123, CD20, CD22, CD30, CD33, CD38, LeY, ROR1, CLL-1, BCMA, et les combinaisons de ceux-ci, de préférence CD19.

3. Composition de cellule CAR pour une utilisation selon la revendication 1 ou 2, dans laquelle ledit agoniste du récepteur S1P est sélectionné parmi mocravimod, siponimod, fingolimod, ozanimod, ponesimod, etrasimod, AKP-11, cenerimod, amiselimod, CBP-307, OPL-307, OPL-002, BMS-986166, SCD-044, BOS-173717, CP-1050, de préférence mocravimod.

4. Composition de cellule CAR pour une utilisation selon la revendication 3, dans laquelle l'agoniste du récepteur S1P est de la formule (I) ou (II) ou (IIa) ou (IIb) suivante : 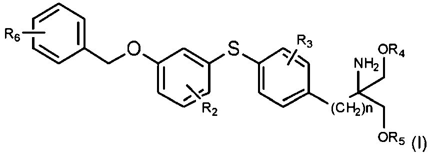 dans laquelle
R₂ est H, halogène, trihalométhyle, alcoxy en C₁₋₄, alkyle en C₁₋₇, phényléthyle ou benzyloxy ;
R₃ est H, halogène, CF₃, OH, alkyle en C₁₋₇, alcoxy en C₁₋₄, benzyloxy, phényle ou alcoxyméthyle en C₁₋₄ ;
chacun parmi R₄ et R₅, indépendamment, est H ou un résidu de formule (a) 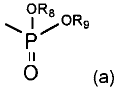
dans laquelle chacun parmi R₈ et R₉, indépendamment, est H ou alkyle en C₁₋₄ facultativement substitué par halogène ;
et n est un nombre entier de 1 à 4 ; et
R₆ est hydrogène, halogène, alkyle en C₁₋₇, alcoxy en C₁₋₄ ou trifluorométhyle,
ou, 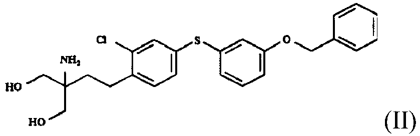
ou les sels pharmaceutiquement acceptables de celui-ci
ou,

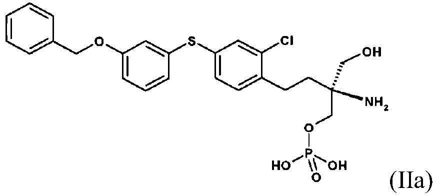

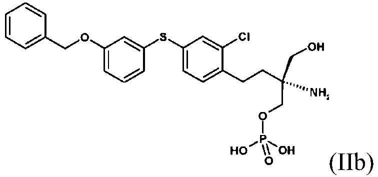

5. Composition de cellule CAR pour une utilisation selon l'une quelconque des revendications 1 à 4, dans laquelle l'agoniste du récepteur S1P est mocravimod, ou un sel pharmaceutiquement acceptable de celui-ci ou un dérivé phosphate de celui-ci.

6. Composition de cellule CAR pour une utilisation selon l'une quelconque des revendications 1 à 5, dans laquelle ladite hémopathie maligne est une leucémie et/ou un lymphome, de préférence sélectionnée parmi le groupe consistant en le lymphome diffus à grandes cellules B (DLBCL), la leucémie myéloïde chronique (CML), la leucémie myéloïde aiguë (AML), la leucémie lymphocytaire chronique (CLL), la leucémie lymphocytaire aiguë (ALL), le lymphome hodgkinien, le lymphome non hodgkinien, le lymphome à cellules du manteau (MCL), le lymphome primaire médiastinal à grandes cellules B (PMBCL) ou le myélome multiple, plus préférentiellement ALL, DLBCL, PMBCL et MCL, encore plus préférentiellement DLBCL.

7. Composition de cellule CAR pour une utilisation selon l'une quelconque des revendications 1 à 6, dans laquelle une quantité efficace de cellules CAR, de préférence de lymphocytes T CAR, est administrée à une posologie d'entre 0,1 x 10⁶ et 6 x 10⁸ cellules immunitaires viables CAR-positives/kg de poids corporel.

8. Composition de cellule CAR pour une utilisation selon l'une quelconque des revendications 1 à 7, dans laquelle lesdites cellules CAR, de préférence des lymphocytes T CAR, sont administrées 2 à 14 jours après l'achèvement de la chimiothérapie lymphodéplétive.

9. Composition de cellule CAR pour une utilisation selon l'une quelconque des revendications 1 à 8, dans laquelle ledit agoniste du récepteur S1P est administré par jour à une posologie d'entre 0,05 mg et 40 mg, de préférence de 0,1 mg à 35 mg, plus préférentiellement de 0,5 mg à 30 mg, encore plus préférentiellement de 1 mg à 15 mg, encore plus préférentiellement de 1,5 mg à 7 mg, encore plus préférentiellement de 2 mg à 5 mg, encore plus préférentiellement d'environ 3 mg ou d'environ 1 mg.

10. Composition de cellule CAR pour une utilisation selon l'une quelconque des revendications 1 à 9, dans laquelle ledit agoniste du récepteur S1P est administré quotidiennement pendant au moins 1, 2 ou 3 mois, ou plus, de préférence à partir d'un jour de début entre 1 et 20 jours avant l'administration de ladite composition comprenant des cellules CAR, plus préférentiellement 11 jours avant une administration de cellules CAR.

11. Composition de cellule CAR pour une utilisation selon l'une quelconque des revendications 1 à 10, dans laquelle ledit agoniste du récepteur S1P est administré en une quantité suffisante pour empêcher les cellules CAR de quitter la moelle osseuse et/ou favoriser une greffe et une persistance de cellules CAR et/ou augmenter l'efficacité d'une thérapie par cellules CAR.

12. Composition de cellule CAR pour une utilisation selon l'une quelconque des revendications 1 à 11, dans laquelle ledit agoniste du récepteur S1P pour une utilisation est administré en une quantité suffisante pour réduire le risque de syndrome de libération de cytokines, en particulier le syndrome systémique de libération de cytokines, chez un sujet recevant des cellules CAR, de préférence autologues ou syngéniques.

13. Agoniste du récepteur S1P pour une utilisation dans la prévention du syndrome de libération de cytokines (CRS) et/ou du syndrome d'activation des macrophages (MAS), et/ou du syndrome de neurotoxicité associée aux cellules effectrices de l'immunité (ICANS) avec une thérapie par cellules CAR (par ex ; thérapie anti-CD19) chez un sujet en ayant besoin, comprenant l'administration d'un agoniste du récepteur S1P ou d'un sel pharmaceutiquement acceptable de celui-ci ou d'un dérivé phosphate de celui-ci, en combinaison avec la thérapie par cellule CAR, au sujet, ce qui empêche ainsi le CRS, et/ou le MAS, et/ou l'ICANS chez le sujet.

14. Modulateur du récepteur S1P selon la revendication 13, dans lequel ladite thérapie par cellules CAR est une thérapie par lymphocytes T CAR, de préférence une thérapie par lymphocytes T CAR anti-CD19.

15. Modulateur du récepteur S1P selon la revendication 13 ou 14, dans lequel ledit agoniste de modulateur du récepteur S1P est mocravimod, ou un sel pharmaceutiquement acceptable de celui-ci, ou un dérivé phosphate de celui-ci.
